# EUROPEAN PATENT APPLICATION

(11) **EP 4 289 941 A1**
(43) Date of publication of application: **13.12.2023**
(21) Application number: 20963382.5
(22) Date of filing: 30.12.2020
(51) Int. Cl.: C12N 5/079, C12N 5/0797, C12N 5/071, C12N 5/0775, C12N 5/0735, C12N 5/10, A61K 35/30, A61P 27/02, A61P 27/06, A61P 27/12, C12Q 1/02

(54) **METHOD FOR REPROGRAMMING CELL**

(30) Priority: 26.11.2020 CN 202011345680
(71) Applicant: MOUCELLION THERAPEUTICS Ltd., Beijing 102206 (CN)
(72) Inventor: LIN, Weifeng, Beijing 102206 (CN)
(74) Representative: Cabinet Becker et Associés
(86) International application number: PCT/CN2020/141524
(87) International publication number: WO 2022/110494

(57) **Abstract**

Provided are a method for reprogramming a cell in the presence of one or more reprogramming factors, a reprogrammed cell obtained by using the method and use thereof, and a kit comprising the reprogramming factors.

## Description

### FIELD OF THE INVENTION

The present disclosure generally relates to a method for reprogramming cells.

### BACKGROUND

Properly functioning corneal endothelial cells (CECs) maintain the transparency and proper fluid levels in the cornea, e.g., the balance between "leakage" of fluid into the stroma and active pumping that continuously operates to move fluid from the stroma to the anterior chamber of the eye.

Corneal endothelial cells have been reported to have little or no capacity to proliferate *in vivo* and thus they cannot be naturally replaced when injured or otherwise lost. In humans, the corneal endothelial cell layer is most densely packed at birth and cell density thereafter decreases rapidly as the eye grows (resulting in the same number of cells covering a larger area). Thereafter, the corneal cell density gradually declines with age, apparently reflecting the gradual loss of cells which are not replaced. As the cell density decreases, each cell spreads out and covers a larger area to maintain the cell layer's barrier and pump functions. However, once the cell density drops too low (lower than about 500 to 1000 cells/mm²), its function would be compromised, resulting in corneal clouding, stromal edema, loss of visual acuity and eventual blindness.

Although different therapeutic methods have been developed, there is a significant need for novel corneal endothelial reconstruction.

### BRIEF SUMMARY OF THE INVENTION

The present disclosure provides a method for reprogramming a first type of cells into a second type of cells, comprising culturing the first type of cells in the presence of a first group of reprogramming factors, wherein the first group of reprogramming factors comprises a glycogen synthases kinase 3 (GSK3) inhibitor, a transforming growth factor-β (TGFβ) inhibitor, and a cyclic AMP inducer.

In certain embodiments, the first group of reprogramming factors may further comprise a basic fibroblast growth factor (bFGF), a DNA methyltransferase inhibitor, a histone methyltransferase inhibitor (e.g. DOT1L inhibitor), a histone deacetylase (HDAC) inhibitor, BMP4, or a combination thereof.

In certain embodiments, the first group of reprogramming factors may be selected from: (a) a GSK3 inhibitor, a TGFβ inhibitor, and a cyclic AMP inducer; (b) a GSK3 inhibitor, a TGFβ inhibitor, a cyclic AMP inducer and bFGF; or (c) a GSK3 inhibitor, a TGFβ inhibitor, a cyclic AMP inducer, a DNA methyltransferase inhibitor, a histone methyltransferase inhibitor (e.g. DOT1L inhibitor) and a histone deacetylase (HDAC) inhibitor.

In certain embodiments, the GSK3 inhibitor may be selected from a group consisting of CHIR99021, LiCl, Li₂CO₃, BIO ((2'Z, 3'E)-6-Bromoindirubin-3'-oxime), TD114-2, Kenpaullone, TWS119, CBM1078, SB216763, 3F8(TOCRIS), AR-A014418, FRATide, indirubin-3'-oxime and L803.

In certain embodiments, the TGFβ inhibitor may be selected from a group consisting of SB431542, Repsox, 616452, LDN193189, A8301, GW788388, SD208, SB525334, LY364947, D4476, SB505124, and Tranilast.

In certain embodiments, the cyclic AMP inducer may be forskolin, IBMX, Rolipram, 8BrcAMP, Prostaglandin E2 (PGE2), NKH477, Dibutyryl Monocyclic Adenylic Acid (DBcAMP), and Sp-8-Br-cAMPs.

In certain embodiments, the DNA methyltransferase inhibitor may be selected from a group consisting of 5-aza-dC, 5-azacytidine, and RG108.

In certain embodiments, the DOT1L inhibitor may be EPZ004777.

In certain embodiments, the HDAC inhibitor may be selected from a group consisting of Valproic acid (VPA), trichostatin A (TSA), vorinostat, depsipeptide, Trapoxin, Depudecin, FR901228, and butyrate.

In certain embodiments, the first type of cells may be somatic cells. In certain embodiments, the somatic cells may be derived from mesoderm, ectoderm or endoderm.

In certain embodiments, the somatic cells may be fibroblasts. In certain embodiments, the fibroblasts may be selected from a group consisting of mouse embryonic fibroblasts (MEFs), mouse tail tip fibroblasts (TTFs), human embryonic fibroblasts (HEFs), human neonatal fibroblasts (HNFs), human adult fibroblasts (HAFs), human foreskin fibroblasts (HFF), and a mixture thereof.

In certain embodiments, the somatic cells may be human exfoliated renal epithelial cells.

In certain embodiments, the first type of cells may be stem cells. In certain embodiments, the stem cells may be selected from a group consisting of human umbilical cord mesenchymal stem cells, human embryonic stem cells, and induced pluripotent stem cells (iPSCs).

In certain embodiments, the second type of cells may be stem cells. In certain embodiments, the stem cells may be neural crest-like cells (NCC-like cells). In certain embodiments, the NCC-like cells may be positive for P75, Hnk1, AP2α, and Sox10.

In certain embodiments, the first type of cells may be cultured in the presence of the first group of reprogramming factors for (a) at least 1, 2, 3, 4, 5, 6, 7, 8, 9, 10, 11, or 12 days or for (b) no more than 20, 19, 18, 17, 16, 15, 14, 13, or 12 days.

The present disclosure provides a method for reprogramming the second type of cells into a third type of cells, comprising culturing the second type of cells in the presence of a second group of reprogramming factors, wherein the second group of reprogramming factors comprises a TGFβ inhibitor and casein kinase 1 inhibitor.

In certain embodiments, the second group of reprogramming factors may further comprise BMP4 and/or a DNA methyltransferase inhibitor. In certain embodiments, the casein kinase 1 inhibitor may be CKI-7. In certain embodiments, the DNA methyltransferase inhibitor may be selected from a group consisting of 5-Azacytidine, 5-aza-dC, and RG108.

In certain embodiments, the third type of cells may be somatic cells. In certain embodiments, the somatic cells may be corneal endothelial cell (CEC)-like cells. In certain embodiments, the CEC-like cells may be positive for ZO-1 and Na⁺/K⁺-ATPase.

In certain embodiments, the second type of cells may be cultured in the presence of the second group of reprogramming factors for (a) at least 1, 2, 3, 4, 5, 6, 7, 8, 9, 10, 11, or 12 days, or for (b) no more than 20, 19, 18, 17, 16, 15, 14, 13, or 12 days.

The present disclosure provides a method of reprogramming a first type of cells into a third type of cells, comprising the following steps: (a) culturing the first type cells in the presence of a first group of reprogramming factors, wherein the first group of reprogramming factors comprises a glycogen synthases kinase 3 (GSK3) inhibitor, a transforming growth factor (TGFβ) inhibitor, and a cyclic AMP inducer; and (b) culturing cells obtained from step (a) in the presence of second group of reprogramming factors, wherein the second group of reprogramming factors comprises a TGFβ inhibitor and a casein kinase 1 inhibitor.

In certain embodiments, the method may further comprise a step of washing cells obtained from step (a), before starting step (b). In certain embodiments, there is no washing step between steps (a) and (b).

The present disclosure provides a method of reprogramming a first type of cells into a third type of cells, comprising a step of culturing the first type of cells in the presence of a first group of reprogramming factors and a second group of reprogramming factors, wherein the first group of reprogramming factors comprises a glycogen synthases kinase 3 (GSK3) inhibitor, a transforming growth factor (TGFβ) inhibitor, and a cyclic AMP inducer, and the second group of reprogramming factors comprises a TGFβ inhibitor and a casein kinase 1 inhibitor.

The present disclosure provides a population of NCC-like cells produced according to the method provided herein.

The present disclosure provides a population of corneal endothelial cell-like cells (CEC-like cells) produced according to the method provided herein.

The present disclosure provides a composition comprising the NCC-like cells or CEC-like cells provided herein.

The present disclosure provides a method for treating a disease or condition associated with dysfunctional or injured corneal endothelial cells, comprising administrating an effective amount of the CEC-like cells provided herein or the composition provided herein to a subject in need thereof. In certain embodiments, the subject may be human.

In certain embodiments, the disease or condition may be selected from a group consisting of Fuch's dystrophy, iridocomeal endothelial syndrome, posterior polymorphous dystrophy, congenital hereditary endothelial dystrophy, age-related macular degeneration (AMD), retinitis pigmentosa, glaucoma, corneal dystrophies, contact lens usage, cataract surgery, and late endothelial failure in cornea transplantation.

The present disclosure provides a kit for reprograming a first type of cells into a second type of cells, wherein the kit comprises a first group of reprogramming factors, which comprises a glycogen synthases kinase 3 (GSK3) inhibitor, a TGFβ inhibitor, and a cyclic AMP inducer.

The present disclosure provides a kit for reprograming a second type of cells into a third type of cells, wherein the kit comprises second group of reprogramming factors, which comprises a TGFβ inhibitor and a casein kinase 1 inhibitor.

The present disclosure provides a kit for reprograming a first type of cells into a third type of cells, wherein the kit comprises: a first group of reprogramming factors which comprises a glycogen synthases kinase 3 (GSK3) inhibitor, a TGFβ inhibitor, and a cyclic AMP inducer; and a second group of reprogramming factors which comprises a TGFβ inhibitor and a casein kinase 1 inhibitor.

The present disclosure provides a method of identifying a drug affecting the effect of NCCs, chemically induced NCC (ciNCCs) or chemically induced CECs (ciCECs), comprising the following steps: administering a drug candidate to NCCs, ciNCCs or ciCECs, and detecting a response of the cells to the drug candidate, thereby identifying the drug.

### BRIEF DESCFRIPTION OF FIGURES

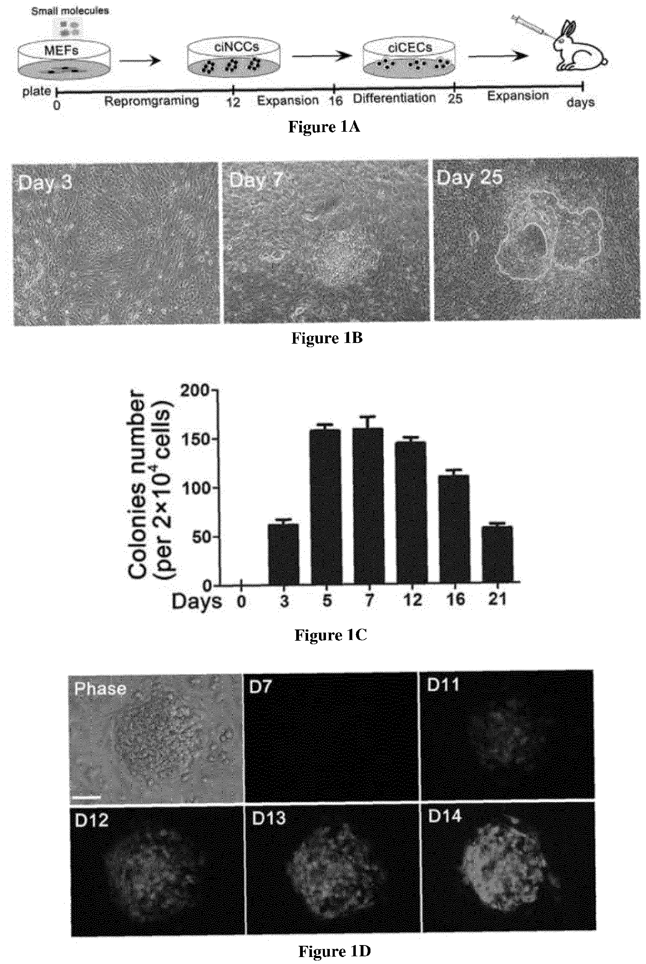

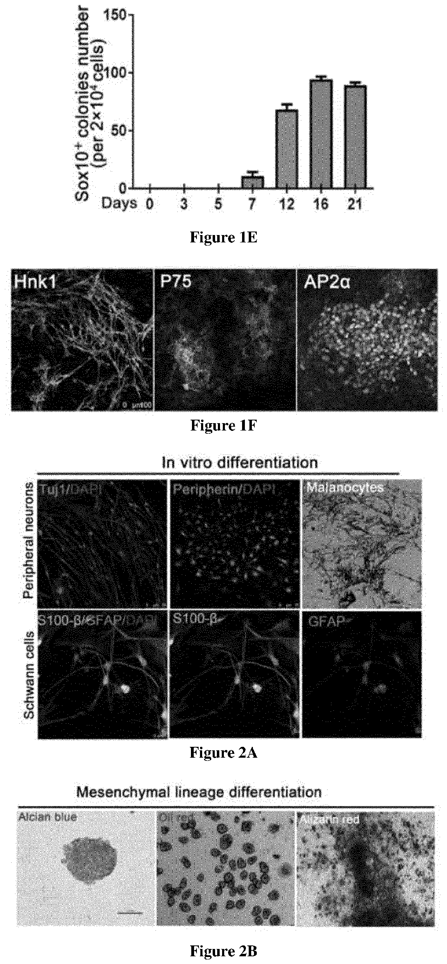

Figure 1 shows conversion of fibroblasts into ciNCCs in a condition with determined chemical compositions. A) illustrates a schematic diagram for induction approach from mouse fibroblasts. B) shows the morphological changes at distinct time points during the induction of neural crest cell (NCC)-like cells. C) shows the numbers of ciNCC colonies generated under indicated conditions. Data are mean ± SD, n = 3 independent experiments. D) shows the images of SOX10⁺ colonies taken by fluorescence microscope *in situ.* E) shows the numbers of SOX10⁺ colonies generated under indicated conditions. Data are mean ± SD, n = 3 independent experiments. F) shows immunostaining of ciNCC markers P75, Hnk1, and AP2α on day 12.
Figure 2 shows the differentiation potential of ciNCCs converted from fibroblasts. A) shows the immunocytochemistry analysis results which show that ciNCCs can differentiated into peripheral neurons as indicated by Tuj 1 and peripherin, and to schwann cells as indicated by S 100β and GFAP. The bright-field images on the right panel show melanocytes differentiated from ciNCCs. B) shows ciNCCs can be further differentiated into chondrocytes, adipocytes and osteocytes as shown by Alcian blue staining, Oil Red O staining and Alizarin Red staining, respectively.

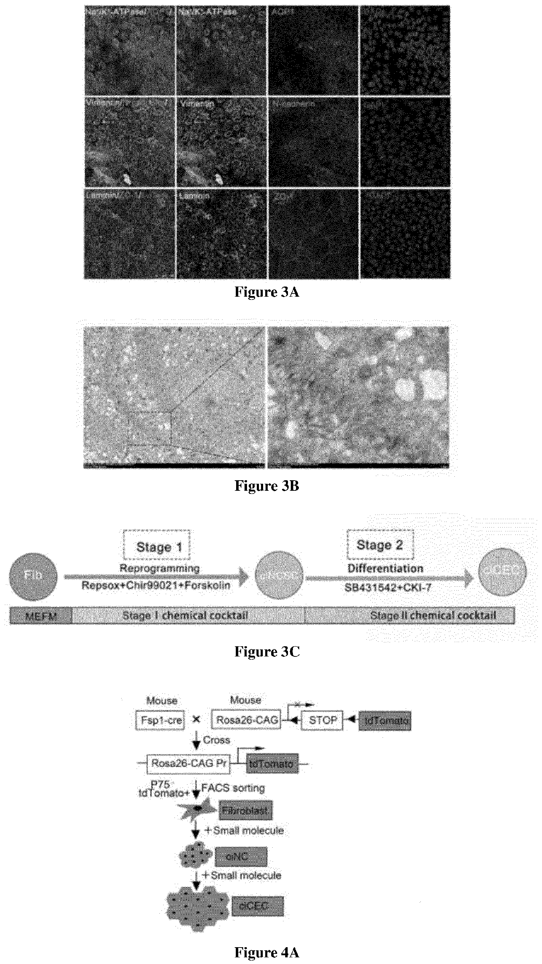

Figure 3 shows ciNCCs can be further differentiated into corneal endothelial cell (CEC)-like cells (or chemically induced CEC, ciCEC). Figure 3A shows immunofluorescence staining of corneal endothelial markers, including Na⁺-K⁺ ATPase, AQP1, Vimentin, N-cadherin, laminin and AQP1. Nuclei are stained with DAPI. Scale bar, 50 µm. Figure 3B shows transmission electron microscope images which show the tight junction of corneal endothelial cells. Figure 3C shows the scheme of a two-step lineage reprogramming strategy by which the corneal endothelial cells are generated functionally from fibroblasts.

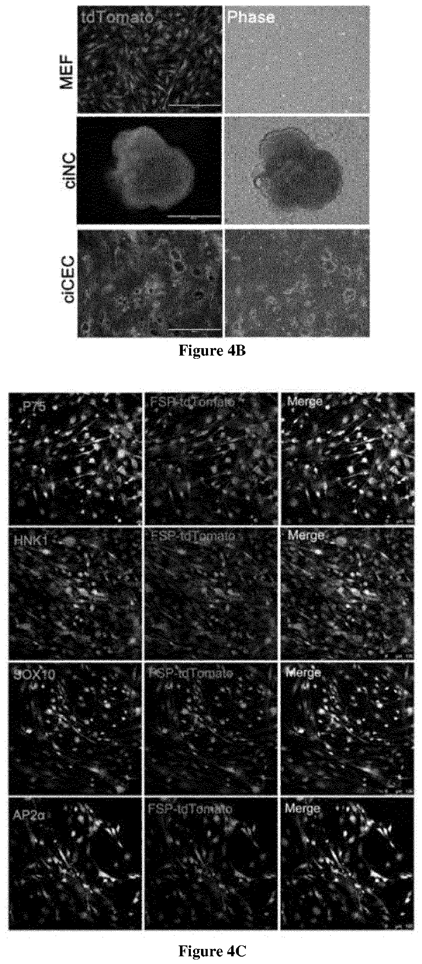

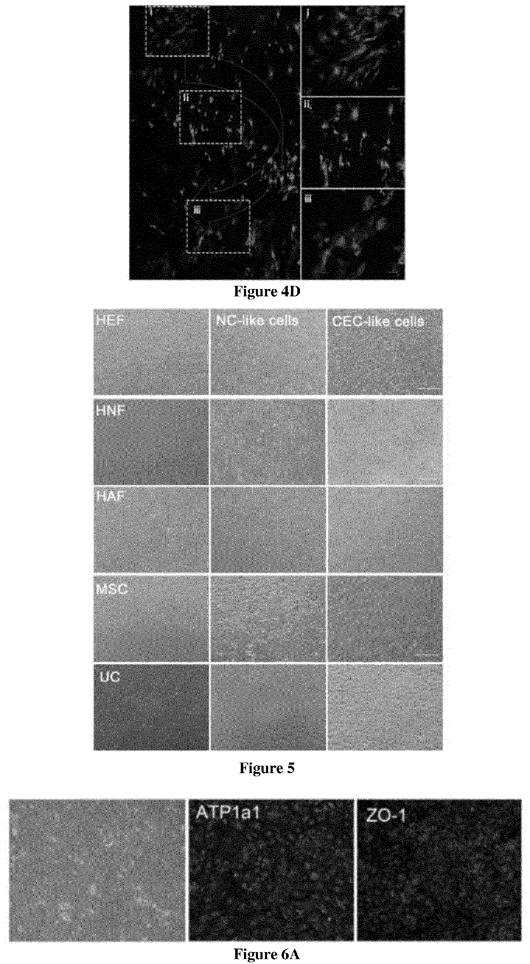

Figure 4 shows the lineage tracing of reprogramming of fibroblasts toward CEC-like cells (or ciCEC). A) shows the schematic diagram of the genetic fate mapping method for tracing the origin of CEC-like cells reprogrammed from Fsp1-Cre:R26R^{tdTomato} MEFs. B) shows the lineage tracing the origin of CEC-like cells reprogrammed from Fsp1-Cre:R26R^{tdTomato} MEFs. C) shows the immunocytochemistry analysis results which show that FSP1-ciNCCs are positive for P75, Hnk1, AP2α, and SOX10. D) shows the representative images for the morphology changes of Fsp1-Cre:R26R^{tdTomato} MEFs and ciCECs induced from these MEFs.
Figure 5 shows generation of CEC-like cells from human different somatic cell types by using small molecules. Human embryonic skin fibroblasts (HEFs), human neonatal fibroblasts (HNFs), human adult fibroblasts (HAFs), human umbilical cord mesenchymal stromal cells (MSCs) and urine cells (UCs).

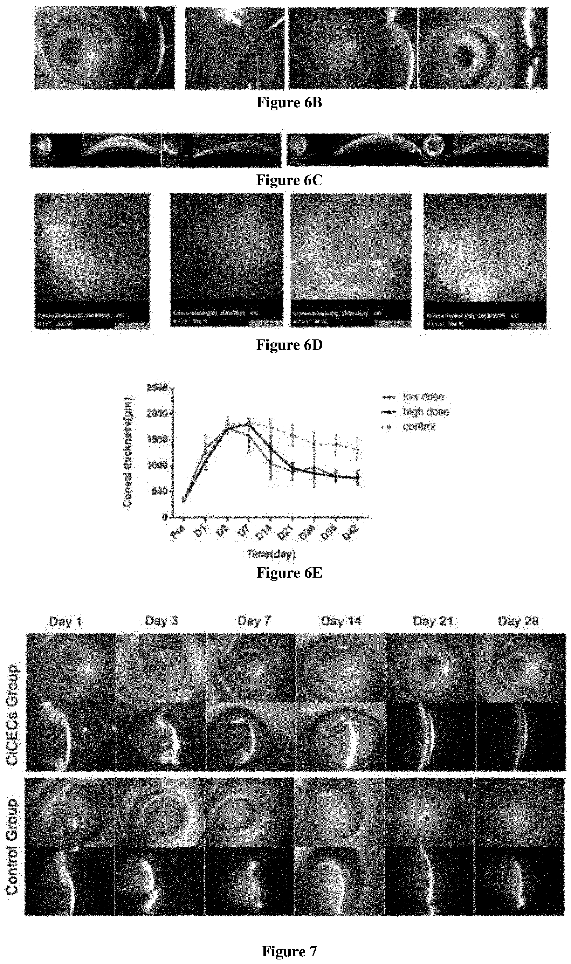

Figure 6 shows clinical observations of the ciCEC group and control group on distinct days in rabbits. A) shows the bright-field images of ciCECs at passage 10 (P10). The ciCECs at passage 10 express Na+-K+ ATPase and ZO-1. B) shows slit-lamp photographs showing that the clarity of corneas of the ciCEC group is significantly improved after injection at day 7 of ciCECs implantation (1^{st} panel), while the corneal opacity and stromal edema are still serious in the untreated control group (3^{rd} panel). C) shows the significant corneal thickness differences between the CEC-like cell group and the control group analyzed by visante OCT. D) shows the confocal microscope images which confirm the full coverage of polygonal cells over the Descemet's membrane in the ciCEC group. E) shows the changes of the corneal thickness during the clinical observation in low dose (1 ×10⁶ cells/ml of ciCECs), high dose (2×10⁶ cells/ml of ciCECs) and PBS-treated control group. In Figures 6B-6D, from left to right: 1^{st} image: ciCEC-treated group, 2^{nd} image: intact contralateral eye, 3^{rd} image: PBS-treated control group, 4^{th} image: normal eye group.
Figure 7 shows the slit-lamp photographs of the ciCEC group and control group on distinct days in rabbits. The slit-lamp photographs show that the clarity of the corneas of the ciCEC group is significantly improved after injection, and the pupils and iris textures can be seen (ciCEC group, upper panel). After only about 14 days, the corneas become clearly transparent, while the corneal opacity and stromal edema are still serious in the control group (control group, upper panel). The lower panels of both ciCEC and control groups show the corneal reflectance detected by slit lamps, and each image in the lower panel corresponds to that in the upper panel.

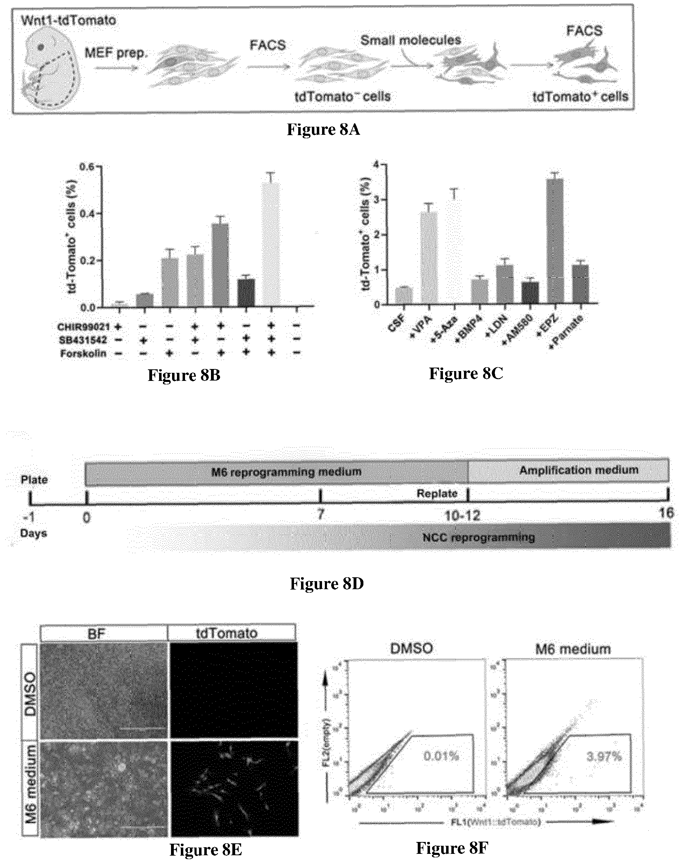

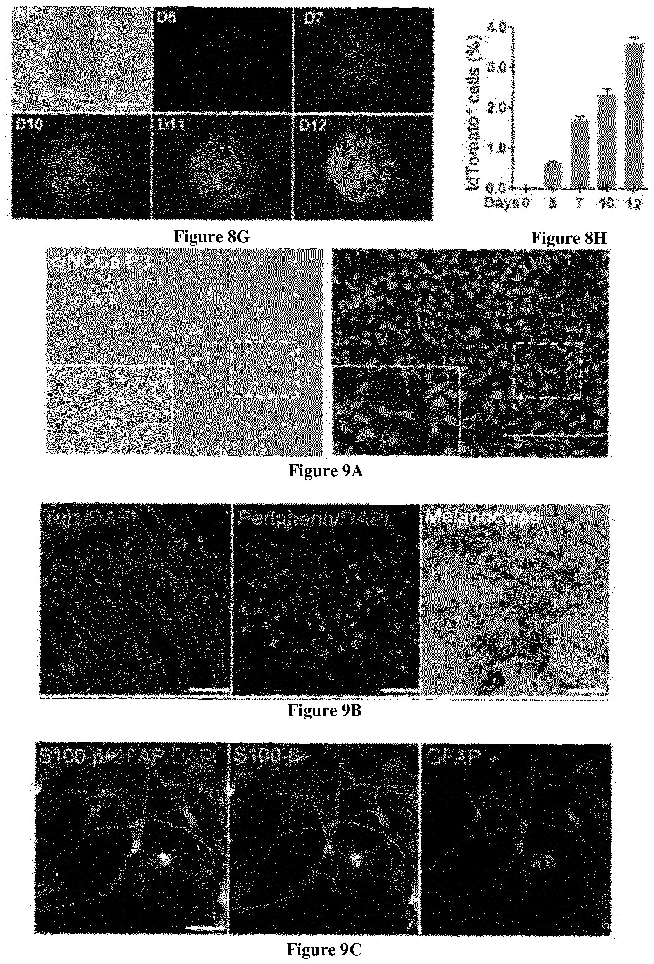

Figure 8 shows conversion of MEFs to ciNCCs induced by small-molecule induction. **A)** Schematic diagram of the reprogramming of NCCs from MEFs. **B)** Effects of individual chemicals on ciNCC generation. Data are means ± SD; *n* = 3 independent experiments. **C)** Enhancement effects of individual chemicals on the generation of ciNCCs. Data are means ± SD; *n* = 3 independent experiments. **D)** Schematic illustration of our strategy for converting MEFs into ciNCCs. **E)** Generation of Wnt1⁺ ciNCCs from MEFs using a cocktail of small molecules. **F)** Quantification of Wnt1⁺ cells in ciNCC clusters induced by candidate cocktails during the combinatorial screening. Independent experiments, n = 3. **G)** Morphological changes on distinct days during the induction process of ciNCCs. Scale bar, 50 µm. **H)** Percentages of Wnt1⁻tdTomato⁺ cells induced by candidate cocktails on distinct days. Independent experiments, n = 3.

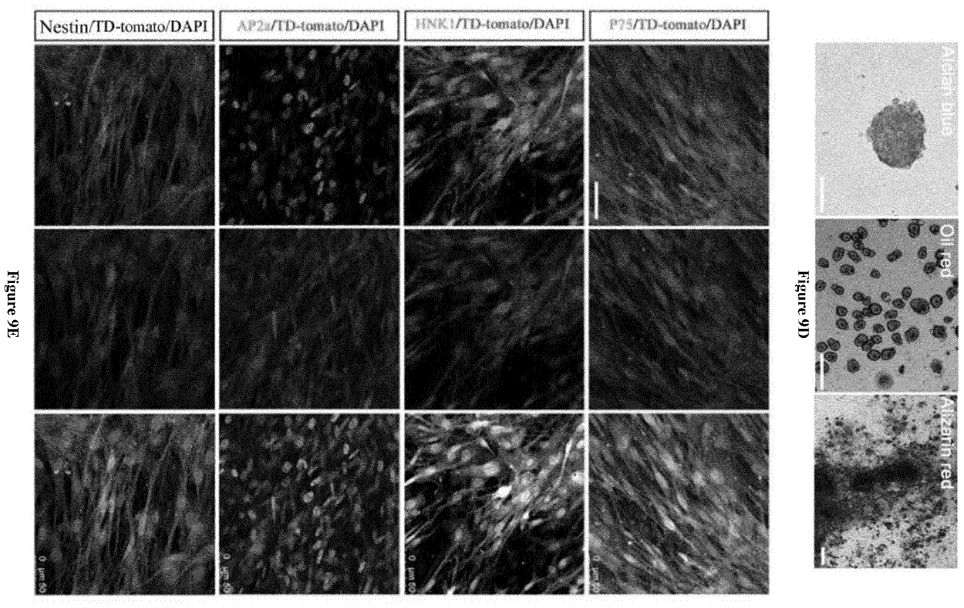

Figure 9 shows the characterization of M6-induced ciNCCs. **A)** Morphology of M6-induced ciNCCs. Scale bar, 400 µm. **B)** Immunostaining images showing that MEF-derived ciNCCs express P75, HNK1, AP2α, and Nestin. Scale bar, 50 µm. **C)** Representative images of differentiated ciNCCs stained with peripheral neuron markers. Scale bars, 50 µm. **D)** Differentiation of ciNCCs into Schwann cells and melanocytes with marker expression. Scale bar, 50 µm. **E)** ciNCCs are differentiated into mesenchymal lineages and further into adipocytes, chondrocytes, and osteocytes. Scale bars, 100 µm.

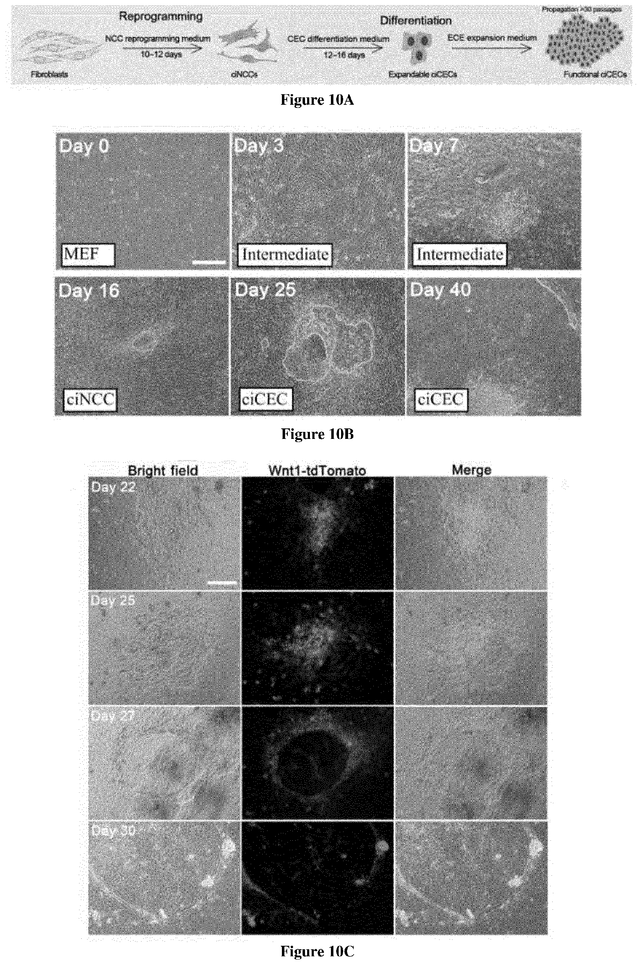

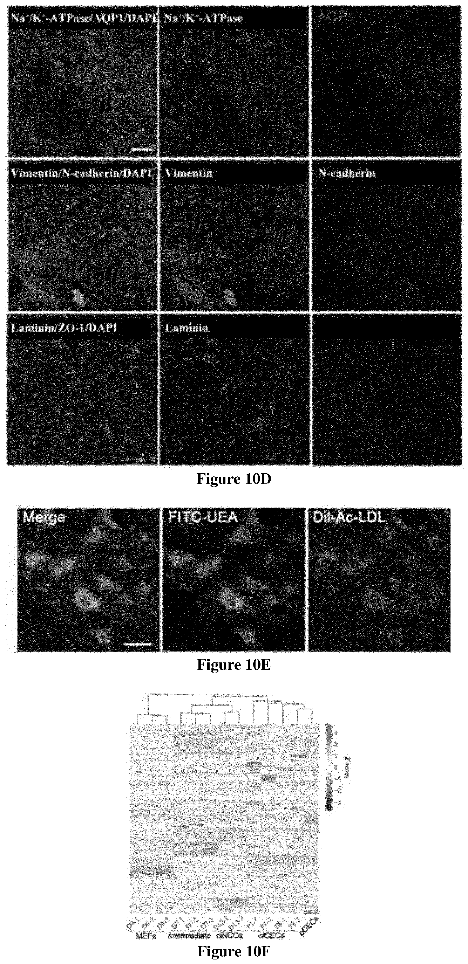

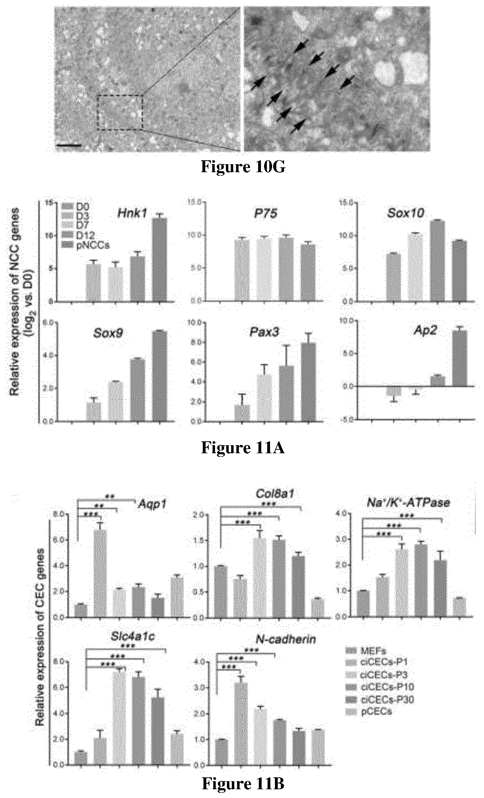

Figure 10 Generation of mouse ciCECs from fibroblasts induced by small-molecule induction. **A)** Schematic diagram for the chemical reprogramming of ciCECs from MEFs. **B)** Bright-field images of initial MEFs, reprogrammed ciNCC colonies, and ciCECs. Scale bar, 400 µm. **C)** Morphological changes on different days during the induction process of ciCECs from Wnt1⁻tdTomato⁺ ciNCCs. Scale bar, 400 µm. **D)** Immunofluorescence staining of ciCECs for the corneal endothelium markers Na⁺/K⁺-ATPase, AQP1, vimentin, N-cadherin, laminin, and ZO-1. Scale bars, 50 µm. **E)** LDL uptake function in ciCECs. Scale bar, 50 µm. **F)** Heatmap of differentially expressed genes among the samples at the indicated time points. The numbers below the heatmap indicate independent biological replicates. Red and blue indicate up- and down-regulated genes, respectively. **G)** TEM of ciCECs showing the tight junctions. Scale bar, 5 µm.

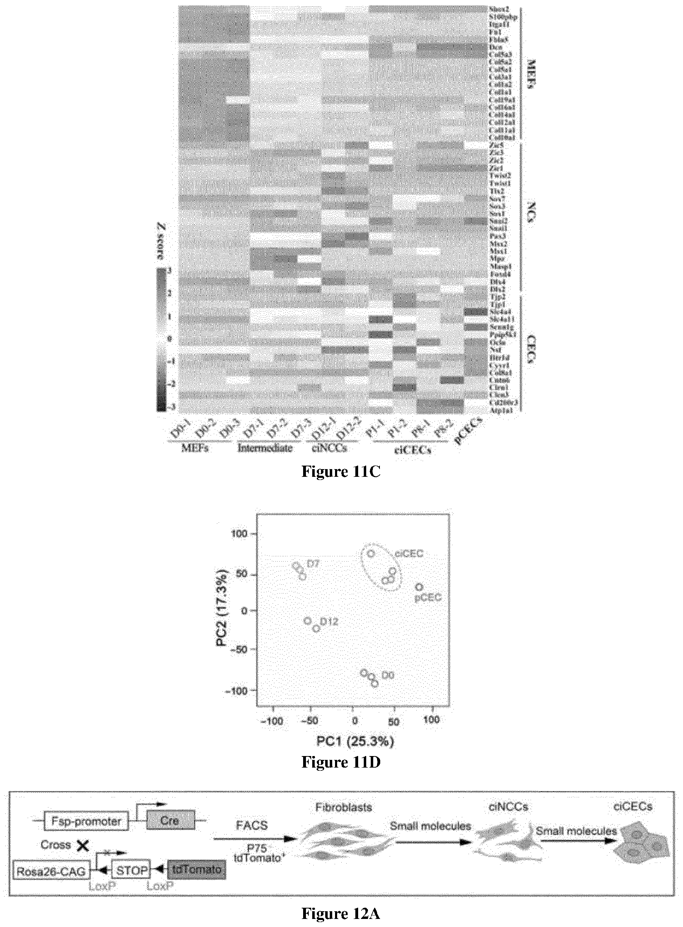

Figure 11 shows gene expression profiling of ciNCCs and ciCECs. **A)** qRT-PCR analysis showing the expression of NC genes at the indicated time points. Gene expression (log2) is normalized to those in MEFs. **B)** qRT-PCR analysis of the expression of the indicated NC cell genes in MEF-derived ciCECs at different passages, MEFs, and pCECs. **C)** Heatmap of differentially expressed genes among samples at the indicated time points. The numbers below the heatmap indicates independent biological replicates (n = 2-3). Red and blue indicate up- and down-regulated genes, respectively. **D)** Principal components analysis of the samples from day 0 (D0), day 7 (D7), and day 12 (D12) of reprogramming, ciCECs, and the control pCECs.

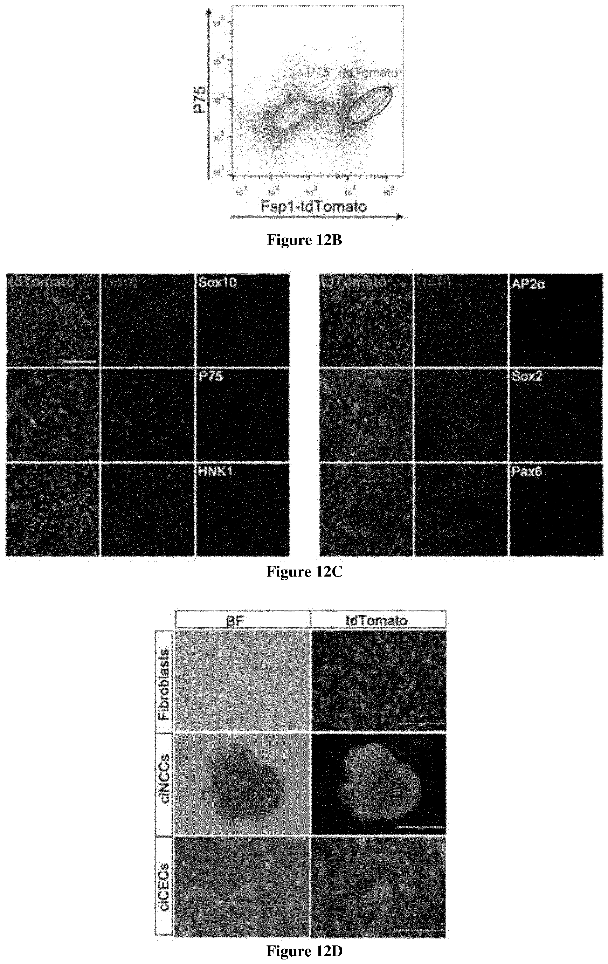

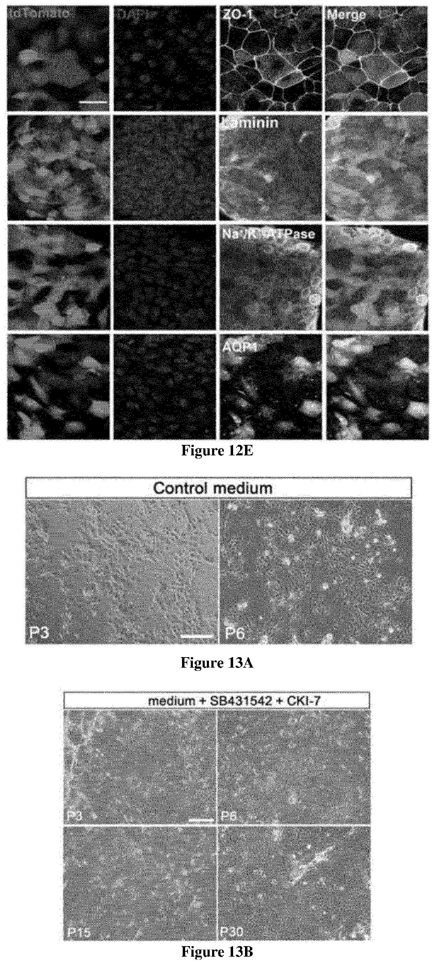

Figure 12 shows the lineage tracing for confirming the induction of ciCECs from fibroblasts. **A)** Schematic diagram illustrating the genetic lineage-tracing strategy. MEFs are obtained by sorting for p75⁻/tdTomato⁺ cells from MEFs derived from E13.5 mouse embryos of the Fsp1-Cre/ROSA26^{tdTomato} background. **B)** FACS result showing the p75⁻/tdTomato⁺ cells from MEFs with a genetic background of *Fsp1-Cre*/*ROSA26*^{tdTomato}. **C)** Immunostaining analysis showing the negative results for Sox10, P75, Olig2, Hnk1, AP2α, Sox2, and Pax6 in the p75⁻/tdTomato⁺ cells. Scale bar, 100 µm. **D)** p75⁻/tdTomato⁺ cell differentiation toward ciNCCs and ciCECs. Scale bars, 400 µm. **E)** Immunostaining analysis showing the positive results for ZO-1, laminin, Na⁺/K⁺-ATPase, and AQP1 in the Fsp1-tdTomato-MEF-derived ciCECs. Scale bar, 50 µm.

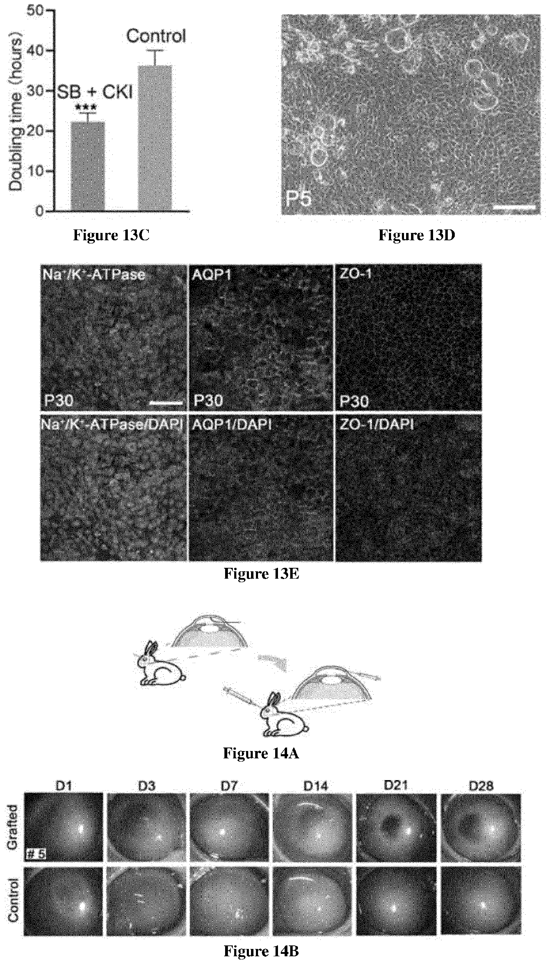

Figure 13 shows small molecules facilitate long-term expansion of ciCECs. **A)** ciCECs were expanded for 3 days in serum-free control medium. Scale bar, 200 µm. **B)** Serial expansion of ciCECs in the serum-free medium with addition of SB431542 and CKI-7. Scale bar, 200 µm. **C)** Average population doubling times (means ± SD, *n* = 3; ***P < 0.001) of the ciCECs cultured in medium with and without SB431542 and CKI-7. **D)** Bright-field image of ciCECs at P5, which were expanded for 3 days in culture condition with addition of SB431542 and CKI-7. Arrows indicate the vacuole-like structures. Scale bar, 50 µm. **E)** These ciCECs at P30 were fixed and stained for Na+/K+-ATPase, AQP1, and ZO-1. Scale bar, 50 µm.

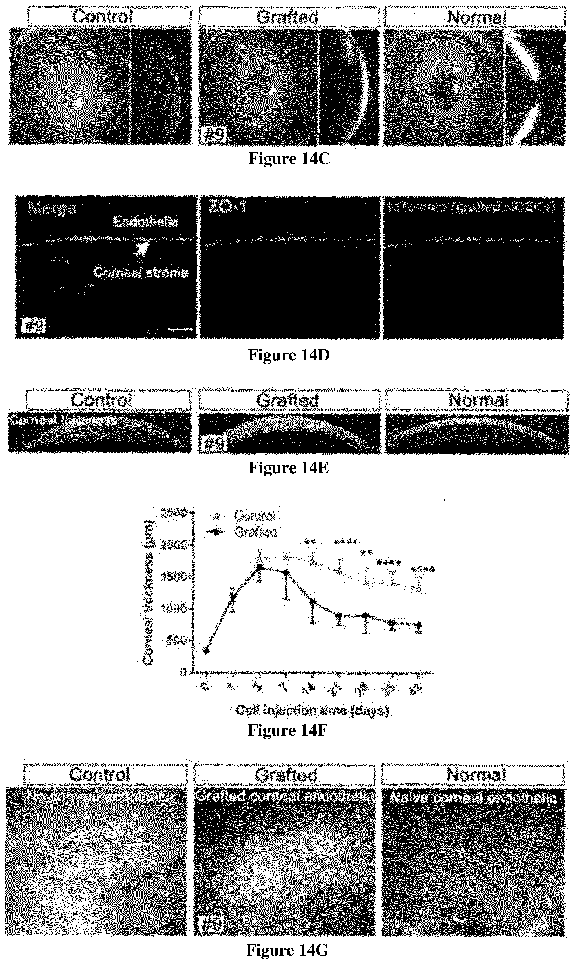

Figure 14 shows transplantation of ciCECs *in vivo.* **A)** Diagram depicting the transplantation of ciCECs with the ROCK inhibitor into model rabbits. **B)** Corneal transparency in the grafted eyes is notably improved after transplantation, while the corneal opacity and stromal oedema are still serious in the untreated controls. **C)** Slit-lamp microscopic images showing that the clarity of the grafted corneas is substantially improved after transplantation, while corneal opacity and stromal oedema remained in the untreated controls. **D)** Immunohistochemistry showing that surviving tdTomato⁺ ciCECs are attached to Descemet's membrane. Scale bar, 100 µm. **E)** Visante OCT showing ameliorated corneal oedema (reduced corneal thickness) in a grafted eye. **F)** Trend in corneal thickness after transplantation. There are significant differences in the corneal thickness and transparency between the untreated controls and the grafted groups. The results are means and SEM for biological replicates (*n* = 9). **G)** Live confocal imaging of corneal endothelia which confirms full coverage of polygonal cells over Descemet's membrane in the grafted eyes.

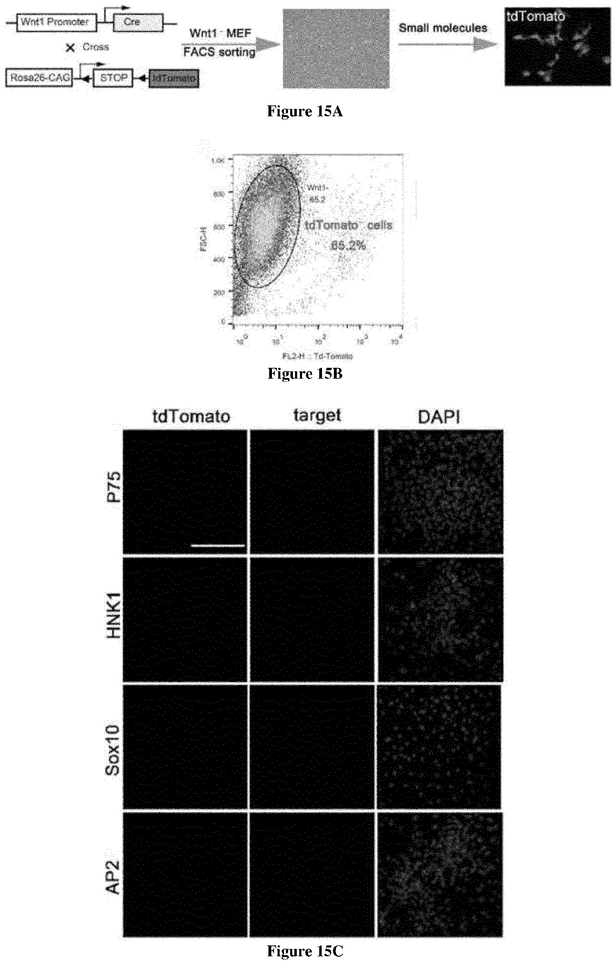

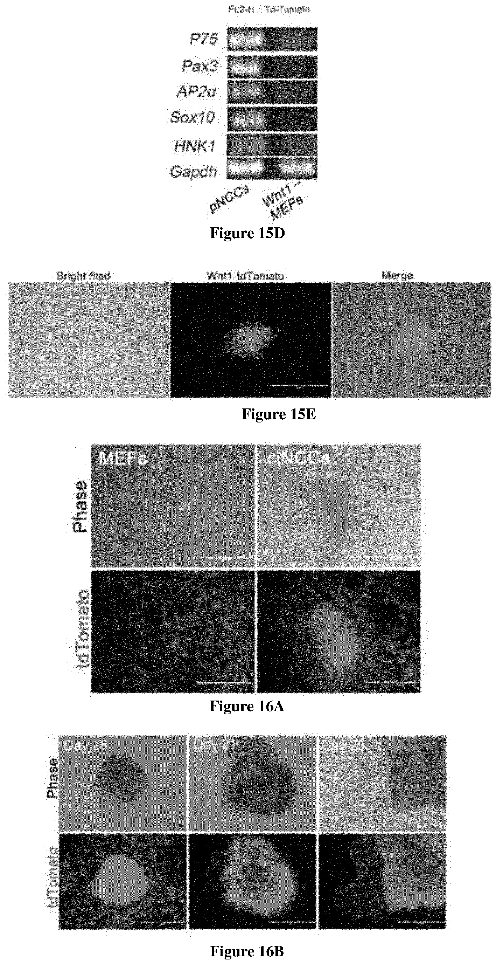

Figure 15 shows the **characterization of Wnt1⁻ MEFs. A)** Schematic diagram illustrating the genetic lineage-tracing strategy. Wnt1⁻ MEFs are obtained by sorting tdTomato⁻cells from MEFs derived from E13.5 mouse embryos of the Wnt1⁻Cre/ROSA26tdTomato background. **B)** FACS sorting result showing the tdTomato cells from MEFs. **C)** Immunostaining analysis showing negative results for P75, HNK1, Sox10, and AP2α in Wnt1⁻MEFs. Scale bar, 100 µm. **D)** RT-PCR analysis showing the expression of the indicated neural crest genes in Wnt1⁻ MEFs and primary NCCs. Gapdh served as the control. **E)** Representative images showing Wnt1⁻ MEF derived ciNCC colonies. Scale bar, 400 µm.

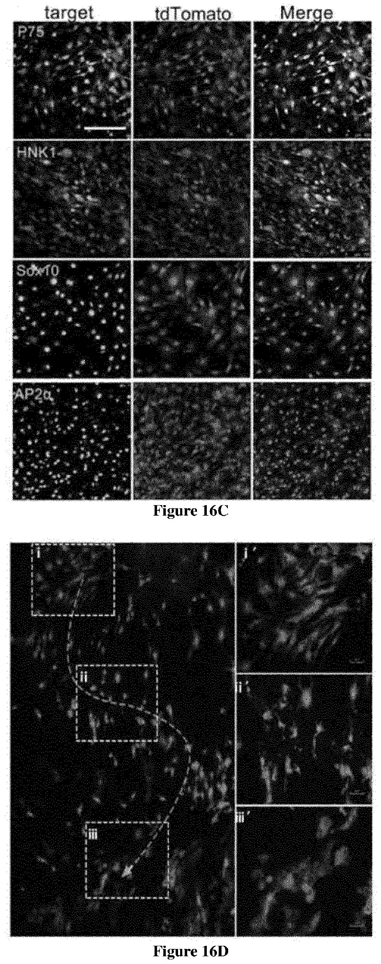

Figure 16 shows M6 converts tdMEFs into ciNCCs. **A)** Morphology of tdMEFs and tdMEF-derived ciNCCs. Scale bars, 400 µm. **B)** Representative images of the differentiation of ciNCCs into ciCECs. Scale bars, 400 µm. **C)** Immunostaining analysis showing the NC cell markers (P75, HNK1, Sox10, and AP2α) expressed by the tdMEF-derived ciNCCs. Scale bar, 50 µm. **D)** The differentiation process of FSP1-tdTomato⁺ ciCECs.

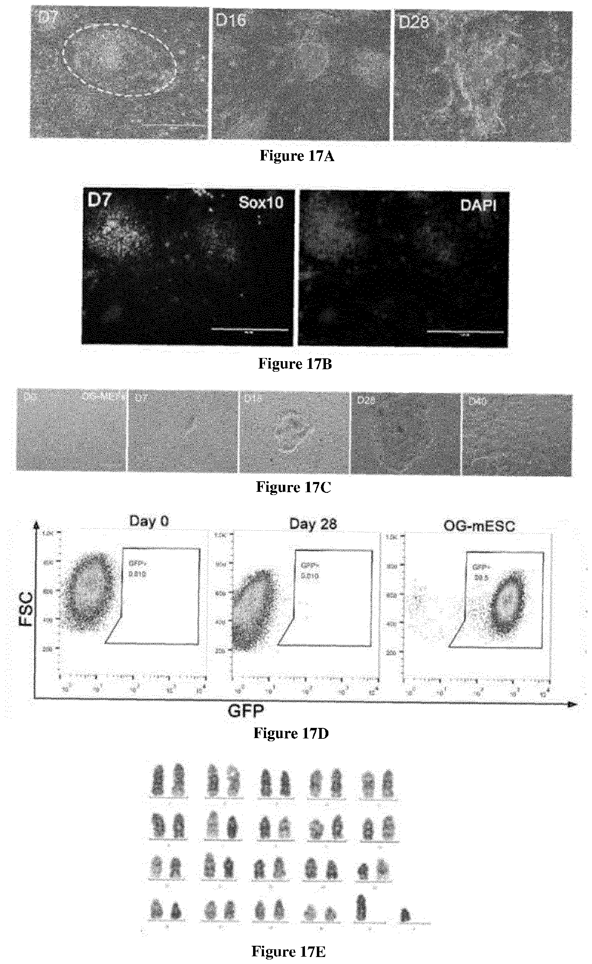

Figure 17 shows generation of ciCECs from fibroblasts induced by small molecules which does not pass through an iPSC stage. **A)** Morphological changes at distinct time points during the induction of ciCECs from MEFs. Scale bar, 400 µm. **B)** Immunofluorescence staining of MEF-derived ciNCC colonies for the NC marker Sox10. Scale bar, 400 µm. **C)** Morphological changes at distinct time points during the induction of ciCECs from OG-MEFs. Scale bar, 400 µm. **D)** Absence of Oct4-GFP-positive cells for ciCEC induction obtained by FACS analysis. **E)** Typical ciCEC karyotype (passage 10).

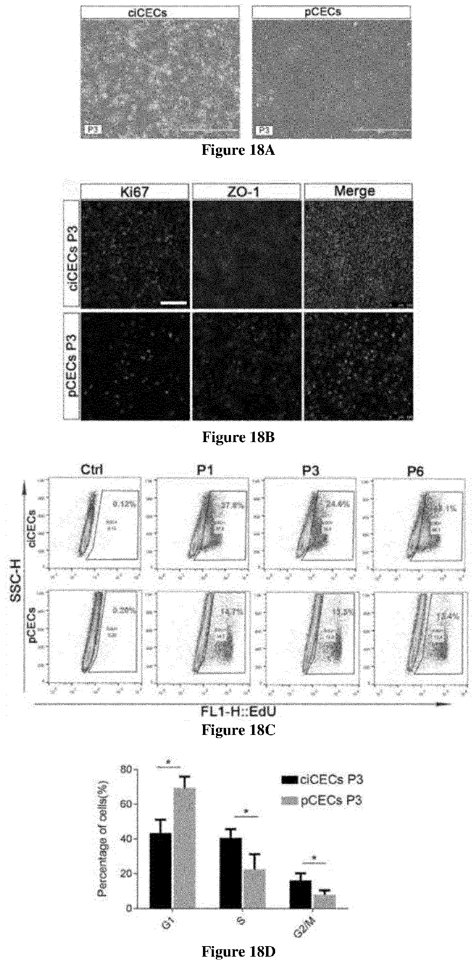

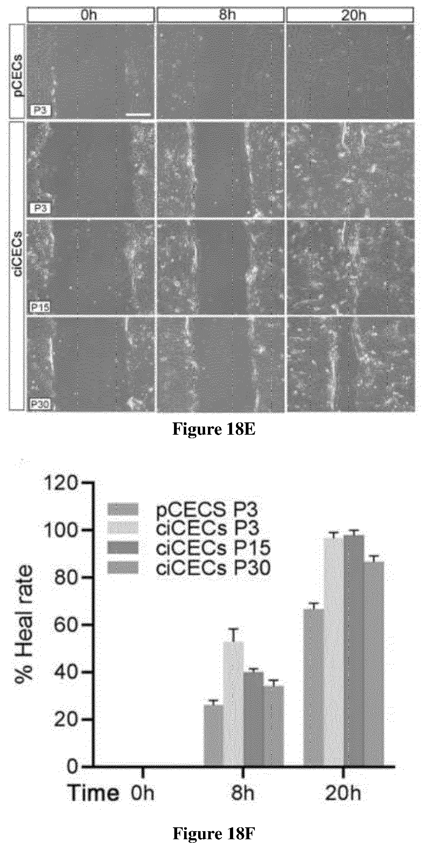

Figure 18 shows the proliferation potential of ciCECs. **A)** Represented images of ciCECs and pCECs at P3. Scale bar, 400 µm. **B)** Immunofluorescence images of the expression of Ki67 and ZO-1 in ciCECs at P3 and pCECs at P3. C) EdU incorporation experiments of ciCECs and pCECs at different passages analyzed by flow cytometry. **D)** Distribution of ciCECs and pCECs in the cell cycle (G1, S, and G2 phases). Scratch ciCECs at P3 and pCECs at P3, observe the migration after 8 h and 20 h and take the photographs. **E)** Scratch wound assays of pCECs at P3 and ciCEC at P3, P15, P30. Migration was observed after 8 h and 20 h. Scale bar, 200 µm. **F)** Quantifications of wound closure are shown.

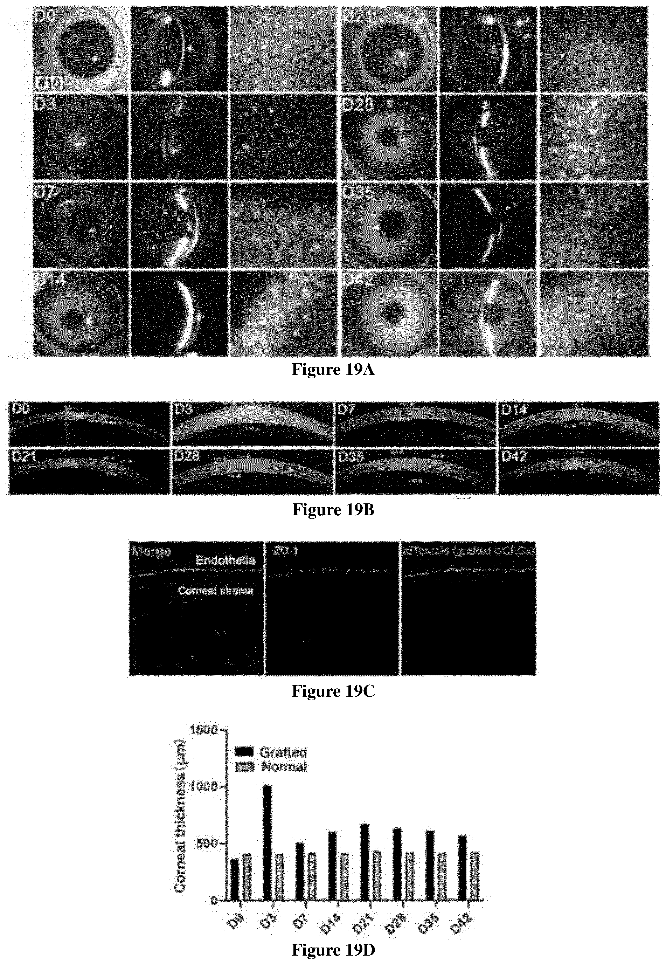

Figure 19 shows observations of the grafted eyes of rabbit models. **A)** Slit-lamp microscopic (middle) and confocal microscope images (right) of grafted eyes in subject #10 on different days at the baseline (before the injection) and after injection of ciCECs supplemented with ROCK inhibitor. **B)** Visante OCT showing the corneal thickness in the ciCEC-grafted eye on different days. **C)** Immunostaining analysis showing surviving tdTomato⁺ ciCECs attached to Descemet's membrane. **D)** Changes in the corneal thickness on different days.

### DETAILED DESCRIPTION OF THE INVENTION

The following description of the disclosure is merely intended to illustrate various embodiments of the disclosure. As such, the specific modifications discussed are not to be construed as limitations on the scope of the disclosure. It will be apparent to one skilled in the art that various equivalents, changes, and modifications may be made without departing from the scope of the disclosure, and it is understood that such equivalent embodiments are to be included herein. All references cited herein, including publications, patents and patent applications, are incorporated herein by reference in their entirety.

### A. General Definitions

The singular terms "a", "an", and "the" include plural referents unless context clearly indicates otherwise. By way of example, reference to "a cell" refers to one or more cells, and reference to "the method" includes reference to equivalent steps and methods disclosed herein and/or known to those skilled in the art, and so forth. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g.," is derived from the Latin exempli gratia, and is used herein to indicate a non-limiting example. Thus, the abbreviation "e.g.," is synonymous with the term "for example".

The term "comprising" or "comprises", as used herein, is used in reference to compositions, methods, and respective component(s) thereof, that are essential to the method or composition, yet open to the inclusion of unspecified elements, whether essential or not.

The term "consisting of" refers to compositions, methods, and respective components thereof as described herein, which are exclusive of any element not recited in that description of the embodiment.

The term "about" or "approximately" means within 20%, preferably within 10%, and more preferably within 5% of a given value or range.

The term "cell" as used herein refers to individual cells, cell lines, or cultures derived from such cells.

The term "reprogram", "reprogramming", "reprogrammed" or equivalents thereof, as used herein, refers to a process that alters or reverses the differentiation status of a cell, either in culture or *in vivo,* than it would have under the same conditions without reprogramming. In other words, under the context of the present disclosure, the "reprogramming" includes differentiation, dedifferentiation and transdifferentiation. The term "differentiation", as used herein, refers to a cellular process by which a less specialized cell becomes a more specialized cell type. In contrast, the term "dedifferentiation" refers to a cellular process in which a partially or terminally differentiated cell reverts to an earlier developmental stage, such as cell having pluripotency or multipotency. In further contrast, the term "transdifferentiation" refers to a cellular process of transforming one differentiated cell type into another differentiated cell type. The term "less-differentiated state" or "less specialized" or "earlier developmental stage", as used herein, is thus a relative term and includes a completely de-differentiated state (or completely de-differentiated) and a partially differentiated state (or partially differentiated). To distinguish from the above mentioned cell development, "undifferentiated cells" are cells capable of differentiating into many directions, i.e., they are capable of differentiating into two or more types of specialized cells. A typical example of an undifferentiated cell is a stem cell.

Cell types pass through various levels of potency during differentiation, such as totipotency, pluripotency and multipotency. The phrase "totipotent stem cells" refer to cells having the ability to differentiate into all cells constituting an organism, such as cells that are produced from the fusion of an egg and sperm cell. Cells produced by the first few divisions of the fertilized egg can also be totipotent. These cells can differentiate into embryonic and extraembryonic cell types. Pluripotent stem cells, such as ES cells, can give rise to any fetal or adult cell type. However, alone they cannot develop into a fetal or adult animal because they lack the potential to develop extraembryonic tissue. Extraembryonic tissue is, in part, derived from extraembryonic endoderm and can be further classified into parietal endoderm (Reichert's membrane) and visceral endoderm (forming a part of the yolk sac). Both parietal and visceral endoderm support the development of the embryo, but do not form embryonic structures. There also are other extraembryonic tissues including extraembryonic mesoderm and extraembryonic ectoderm. As used herein, "pluripotent stem cells" or cells with "pluripotency" or equivalency refer to a population of cells that can differentiate into all three germ layers (e.g., endoderm, mesoderm and ectoderm). Pluripotent cells express a variety of pluripotent cell-specific markers, have cell morphology characteristics of undifferentiated cells (i.e., compact colonies, high ratio of nucleus to cytoplasm, and prominent nucleoli) and form teratomas when introduced into an immunocompromised animal, such as a SCID mouse. The teratomas typically contain the cell or tissue characteristics of all three germ layers. One of ordinary skill in the art can assess these characteristics by using techniques commonly used in the art. See, e.g., Thomson et al., Science 282:1145-1147 (1998). Pluripotent cells are capable of proliferating in cell culture and differentiating towards a variety of lineage-restricted cell populations that exhibit multipotent properties. Multipotent stem cells or cells with multipotency or equivalency have higher differentiation relative to pluripotent stem cells, but are not terminally differentiated. Pluripotent stem cells therefore have a higher potency than multipotent stem cells.

### B. Reprogramming cells

In one aspect, the present disclosure provides a method for reprogramming a first type of cells into a second type of cells, comprising a step of culturing the first type of cells in the presence of first group of reprogramming factors, wherein the first group of reprogramming factors comprises a glycogen synthases kinase 3 (GSK3) inhibitor, a transforming growth factor-β (TGFβ) inhibitor, and a cyclic AMP inducer.

In some embodiments, the step of culturing the first type of cells in the presence of the first group of reprogramming factors may lead to the conversion of at least 50%, 55%, 60%, 65%, 70%,75%, 80%, 85%, 86%, 87%, 88%, 89%, 90%, 91%, 92%, 93%, 94%, 95%, 96%, 97%, 98%, 99%, or more of the cells into the second type of cells.

In certain embodiments, the first type of cells may be mammalian (e.g. human or mouse) cells that are derived from mesoderm, ectoderm or endoderm.

The ectoderm, mesoderm, and endoderm are the three germ layers formed during the embryo development with the mesoderm as the middle layer, the ectoderm as the outside layer and the endoderm as the inside layer. The mesoderm forms mesenchyme, mesothelium, non-epithelial blood cells and coelomocytes, that constitute muscle (smooth and striated), bone, cartilage, connective tissue, adipose tissue, circulatory system, lymphatic system, dermis, genitourinary system, serous membranes, and notochord. The endoderm forms pharynx, esophagus, stomach, small intestine, colon, liver, pancreas, bladder, epithelial parts of the trachea and bronchi, lungs, thyroid, and parathyroid. The ectoderm forms surface ectoderm, neural crest, and neural tube, wherein the surface ectoderm develops into epidermis, hair, nails, lens of the eye, sebaceous glands, cornea, tooth enamel, the epithelium of mouth and nose; the neural crest of the ectoderm develops into peripheral nervous system, adrenal medulla, melanocytes, facial cartilage, and dentin of teeth; and the neural tube of the ectoderm develops into brain, spinal cord, posterior pituitary, motor neurons, and retina.

In certain embodiments, the first type of cells may be somatic cells. As used herein, the term "somatic cell" refers to any cell other than germ line cells (e.g., sperms and ova, and the cells from which they are made (gametocytes)) and undifferentiated stem cells. Internal organs, skin, bones, blood and connective tissue are all made up of somatic cells. The somatic cells may be any type of somatic cells, of any origin. For example, the somatic cells may include, but are not limited to fibroblast cells, epithelial cells, sertoli cells, endothelial cells, granulosa epithelial, neurons, pancreatic islet cells, epidermal cells, hepatocytes, hair follicle cells, keratinocytes, hematopoietic cells, melanocytes, chondrocytes, lymphocytes (B and T lymphocytes), erythrocytes, macrophages, monocytes, mononuclear cells, cardiac muscle cells, and other muscle cells.

In certain embodiments, the first type of cells may be mesodermal cells. The markers, such as CD56 and APJ, of the mesodermal cells are known to the art. In certain embodiments, the mesodermal cells may include fibroblast cells, epithelial cells, leucocytes, adipocytes and keratinocytes. In certain embodiments, the first type of cells may be fibroblasts, including but not limited to, mouse embryonic fibroblasts (MEFs), mouse neonatal fibroblasts (MNFs), mouse tail tip fibroblasts (TTFs), human embryonic skin fibroblasts (HEFs), human neonatal fibroblasts (HNF), human adult fibroblasts (HAF), human embryonic lung fibroblasts, human foreskin fibroblasts (HFF), and the mixture thereof.

The fibroblast cells may be obtained from any suitable source, for example from commercial source or from various organ or skin tissues. Preferred fibroblasts are lung fibroblasts, foreskin fibroblasts, and adult dermal fibroblasts. In certain embodiments, the fibroblasts may be obtained from a patient, for example by skin biopsy (e.g., Reprogramming of human somatic cells to pluripotency with defined factors. George Q. Daley et al. Nature 2008; A method for the isolation and serial propagation of keratinocytes, endothelial cells, and fibroblasts from a single punch biopsy of human skin, Normand et al. In Vitro Cellular & Developmental Biology - Animal, 1995).

In certain embodiments, the first type of cells may be epithelial cells, such as human exfoliated renal epithelial cells. The epithelial cells can be obtained from urine samples.

In certain embodiments, the first type of cells may be leucocytes. The leucocytes can be obtained from blood samples. The leucocytes may be white blood cells that can be usually classified into their subgroups (lymphocytes, monocytes, neutrophils, eosinophils and basophils) by the difference of RF signal intensity (changes in electric impedances at high-frequency), DC signal intensity (changes in direct current caused by the difference in the electroconductivity between the suspended particles and the liquid medium in which the particles are suspended), intensity of fluorescence, intensity of scattered light, absorbance, depolarization of scattered light or the like (see US5618733A).

In certain embodiments, the first type of cells may be adipocytes or keratinocytes. The adipocytes and keratinocytes can also be easily derived by skin biopsy or plucked hair (isolation and cultivation of human keratinocytes from skin or plucked hair for the generation of induced pluripotent stem cells, Belmonte et al. Nature Protocols 2010).

In other embodiments, the first type of cells may be adult cells. As used herein, the term "adult cells" refers to cells found throughout the body after embryonic development. In certain embodiments, the first type of cells may be stem cells, such as embryonic stem cells, induced pluripotent stem cells (iPSCs) and adult stem cells, including, but not limited to hematopoietic stem cells, vascular endothelial stem cells, cardiac stem cells, muscle-derived stem cells, mesenchymal stem cells, epidermal stem cells, adipose-derived stem cells, intestinal stem cells, neural stem cells, renal epithelium stem cells, urothelial stem cells, and hepatic stem cells.

In some embodiments, the stem cell refers to an undifferentiated cell which is capable of proliferating and giving rise to more progenitor cells having the ability to generate a large number of mother cells that can in turn give rise to differentiated, or differentiable daughter cells. The stem cells can divide asymmetrically, with one daughter cell retaining the stem state and the other expressing some distinct other specific function and phenotype. Alternatively, some of the stem cells in a population can divide symmetrically into two stems, thus maintaining some stem cells in the population as a whole, while other cells in the population give rise to differentiated progeny only. The daughter cells themselves can be induced to proliferate and produce progeny that subsequently differentiate into one or more mature cell types, while also retaining one or more cells with parental developmental potential. In other embodiments, the term "stem cell" refers to a subset of progenitors that have the capacity or potential, under particular circumstances, to differentiate to a more specialized or differentiated phenotype, and that retains the capacity, under certain circumstances, to proliferate without substantial differentiation. In one embodiment, the term stem cell refers generally to a naturally occurring mother cell whose descendants (progeny) usually specialize in different directions by differentiation, e.g., by acquiring completely individual characters, as occurs in progressive diversification of embryonic cells and tissues. A differentiated cell may derive from a multipotent cell which itself is derived from a multipotent cell, and so on. Though each of these multipotent cells may be considered as stem cells, the range of cell types each multipotent cell can give rise to may vary considerably. In many biological instances, stem cells are also "multipotent", because they can produce progeny of more than one distinct cell type, but this is not required for "stem-ness". Self-renewal is the other classical part of the stem cell definition. In theory, self-renewal can occur by either of two major mechanisms.

The term "embryonic stem cell" is used to refer to the pluripotent stem cells of the inner cell mass of the embryonic blastocyst (see U.S. Pat. Nos. 5,843,780, 6,200,806, which are incorporated herein by reference). The distinguishing characteristics of an embryonic stem cell define an embryonic stem cell phenotype. Accordingly, a cell would have the phenotype of an embryonic stem cell, if it possesses one or more of the unique characteristics of an embryonic stem cell which can distinguish the cell from other cells. Exemplary distinguishing embryonic stem cell characteristics include, without limitation, gene expression profile, proliferative capacity, differentiation capacity, karyotype, responsiveness to particular culture conditions, and the like.

The term "adult stem cell" or "ASC" is used to refer to any multipotent stem cell derived from non-embryonic tissues, including fetal, juvenile, and adult tissue. Adult stem cells have been isolated from a wide variety of adult tissues including blood, bone marrow, brain, olfactory epithelium, skin, pancreas, skeletal muscle, and cardiac muscle. Each of these stem cells can be characterized based on gene expression, factor responsiveness, and morphology in culture. As indicated above, the stem cells have been found resident in virtually every tissue. Accordingly, it is appreciated from the technology described herein that stem cell populations can be isolated from virtually any animal tissue.

In certain embodiments, the first type of cells may be mesenchymal stem cells, mesenchymal stromal cells, human embryonic stem cells or induced pluripotent stem cells (iPSCs).

As used herein, the terms "iPS cells", "iPSCs" and "induced pluripotent stem cells" can be used interchangeably and refer to a pluripotent cell artificially derived (e.g., induced by complete or partial reversal) from a differentiated somatic cell (i.e. from a non-pluripotent cell). A pluripotent cell can differentiate to a cell of all three developmental germ layers.

Mesenchymal stem cells (MSCs) or mesenchymal stromal cells are adult stem cells traditionally found in the bone marrow. However, the mesenchymal stem cells can also be isolated from other tissues including cord blood, peripheral blood, fallopian tube, and fetal liver and lung. MSCs can differentiate into a variety of cell types, including osteoblasts (bone cells), chondrocytes (cartilage cells), myocytes (muscle cells) and adipocytes (fat cells which give rise to marrow adipose tissue).

In certain embodiments, the first type of cells may be ectodermal cells. In certain embodiments, the first type of cells may be endodermal cells.

In certain embodiments, the second type of cells may be transdifferentiated from the first type of cells. For example, mesodermal cells may be reprogrammed into ectodermal cells. In certain embodiments, the second type of cells may be ectodermal cells. In certain embodiments, the multipotent stem cells may be neural crest cells (NCCs) or NCC-like cells. In certain embodiments, the second type of cells may be neural crest stem cells (NCCs) or NCC-like cells.

"Neural crest stem cells" or "NCCs" generally refer to a neural progenitor cell having the developmental potential to produce pigmented cells co-expressing the melanosome marker, and HMB45. Neural crest stem cells may be identified by expression of markers identified herein and known in the art. In order to distinguish the chemically induced NCCs (ciNCC) provided herein and primary NCCs, the resulting ciNCCs are named as neural crest cell-like cells (NCC-like cells). In some embodiments, the resulting NCC-like cells exhibit one or more biomarkers consistent with the primary NCC phenotype. In some embodiments, the resulting NCC-like cells lack or have lower expression of one or more biomarkers (e.g., PAX6) consistent with the NCC phenotype. Exemplary NC biomarkers, the presence of which is consistent with the NCC phenotype, can include Nestin, SOX10, SOX9, HNK-1, P75 (NGFR), AP2α, PAX3, PAX7, SNAI2, Snail, Twistl, Krox20, CD271, FoxD3, AN2 and Ki67, and/or at least one pluripotency marker NANOG, ZNF206, or OCT4. In some embodiments, the NC biomarkers may comprise P75, Hnk1, AP2α, and/or SOX10. In embodiments, the expression of NC biomarkers may be increased over 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 600, 700, 800, 900, 1000, 1100, 1200, 1300, 1400, 1500, 1600, 1700, 1800, 1900, 2000 folds or more, relative to the first type of cells from which the NCC-like cells are derived.

In certain embodiments, the first type of cells may be fibroblasts and the second type of cells may be NCC-like cells. In certain embodiments, the first type of cells may be mouse embryonic fibroblasts (MEFs) and the second type of cells may be NCC-like cells. In certain embodiments, the first type of cells may be mouse tail tip fibroblasts (TTFs) and the second type of cells may be NCC-like cells. In certain embodiments, the first type of cells may be human embryonic skin fibroblasts (HEFs) and the second type of cells may be NCC-like cells. In certain embodiments, the first type of cells may be human embryonic lung fibroblasts, human foreskin fibroblasts (HFF) and the second type of cells may be NCC-like cells. In certain embodiments, the first type of cells may be human neonatal fibroblasts (HNF) or human adult fibroblasts (HAF), and the second type of cells may be NCC-like cells.

In certain embodiments, the first type of cells may be epithelial cells (e.g. human exfoliated renal epithelial cells) and the second type of cells may be NCC-like cells. In certain embodiments, the first type of cells may be leucocytes (e.g. lymphocytes, monocytes, neutrophils, eosinophils or basophils) and the second type of cells may be NCC-like cells. In certain embodiments, the first type of cells may be adipocytes and the second type of cells may be NCC-like cells. In certain embodiments, the first type of cells may be keratinocytes and the second type of cells may be NCC-like cells. In certain embodiments, the first type of cells may be MSCs, human embryonic stem (ES) cells or iPSCs and the second type of cells may be NCC-like cells.

In certain embodiments, the second type of cells may be certain types of multipotent stem cells reprogrammed from another type of stem cells (the first type of cells), such as mesenchymal stem cells, human embryonic stem cells or induced pluripotent stem cells (iPSCs).

Expression of the markers may be detected by any method known in the art, including but not limited to, Western Blotting, mRNA amplification-based methods (e.g., PCR, isothermal amplification, etc., which may include reverse transcription and may be applied to detect expression from single cells or multiple cells), Northern blotting, immunostaining, etc. Additionally, expression of said markers may be inferred by expression of a reporter construct (such as a fluorescent protein whose expression may be visually detected, an antibiotic resistance gene whose expression may be detected by cell survival in the presence of the antibiotic, etc.) under the control of a genetic element that confers cell type specific expression, such as the promoter of one of the foregoing markers or a fragment thereof. Exemplary reporter constructs may comprise the pOCT4-GFP and pOCT4-LUC genes which drive expression of GFP and luciferase, respectively, in ES cells. The expression of either of GFP and luciferase is readily detectable using conventional methodologies. Further methods of detecting marker expression that may be used are known in the art. See, generally, Ausubel, Current Protocols in Molecular Biology (Current Protocols, 1988); Ausubel et al., Short Protocols in Molecular Biology (Current Protocols; 5th Edition, 2002); Sambrook et al., Molecular Cloning: A Laboratory Manual (Cold Spring Harbor Laboratory Press, 3rd edition, 2001); Sambrook et al., The Condensed Protocols from Molecular Cloning: A Laboratory Manual (Cold Spring Harbor Laboratory Press, 2006), each of which is incorporated by reference herein in its entirety.

Any cell culture system known in the art can be used in the present disclosure. In certain embodiments, adherent culture system may be used in the methods of the present disclosure. The term "adherent culture" refers to a cell culture system whereby cells are cultured on a solid surface, which may in turn be coated with a substrate. The cells may or may not tightly adhere to the solid surface or to the substrate. The substrate for the adherent culture may further comprise, for example, any one or combination of polystyrene, polyester, polycarbonate, poly(N-isopropylacrylamide), polyomithine, laminin, polylysine, purified collagen, gelatin, cellulose, extracellular matrix, fibronectin, tenacin, vitronectin, poly glycolytic acid (PGA), poly lactic acid (PLA), poly lactic-glycolic acid (PLGA), matrigel, hydroxyapatite, and amniotic membrane.

In certain embodiments, suspension culture can be used in the method of the present disclosure. The term "suspension culture" as used herein refers to the culture of cells such that the cells do not adhere to the solid support or the culture vessel. To transfer cells into a suspension culture, they are for example removed from the culture receptacle by a cell scraper and transferred to sterile low attachment plates containing culture medium, which do not allow adhesion of the cells to the surface of the plate. Thus, the cells are cultured in suspension without adherence to a matrix or the bottom of the dish.

Media suitable for culturing cells may be any media suitable for growing a certain cell type in a dish. The media includes, such as Ham's F 10 (Sigma), Ham's F 12 medium, Minimal Essential Medium (MEM), (Sigma), RPMI-1640 (Sigma), and Dulbecco's Modified Eagle's Medium (DMEM), Sigma), IMDM medium, Medium 199, Eagle's Minimum Essential Medium (EMEM), aMEM medium, Fischer's medium, Neurobasal medium (Life Technologies Corporation), and mixtures of these. In addition, any of the media described in Ham et al., Meth. Enz. 58:44 (1979), Barnes et al., Anal. Biochem. 102:255 (1980), U.S. Pat. No. 4,767,704; 4,657,866; 4,927,762; 4,560,655; or 5,122,469; WO 90/03430; WO 87/00195; or U.S. Pat. Re. 30,985 may be used as the culture media. Any of these media may be supplemented as necessary with salts (such as sodium chloride, calcium salt, magnesium salt, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), glucose or an equivalent energy source, albumin, insulin, transferrin, selenium, fatty acids, 2-mercaptoethanol, thiol glycerol, lipids, amino acids, L-glutamine, non-essential amino acids, vitamins, growth factors, low-molecular-weight compounds, antioxidants, pyruvic acid, cytokines and the like as necessary. Any other necessary supplements may also be included at appropriate concentrations that would be known to those skilled in the art. The culture conditions, such as temperature, pH, and the like, are those previously used for cell culture, and will be apparent to the ordinarily skilled artisan.

In the process of cell reprogramming described herein, cells can be cultured in a basic medium supplemented with one or more reprogramming factors described herein. For example, a basic medium may contain DMEM/F12/Glutamax (GIBCO), 10% KnockOut Serum Replacement(KSR) (GIBCO), 1% NEAA (GIBCO), 10% FBS (GIBCO), and 0.1 mM 2-mercaptoethanol (GIBCO) before addition of the first group of reprogramming factors. For another embodiment, a basic medium may contain DMEM/F12/Glutamax (GIBCO), 0.075% bovine serum albumin (BSA) (GIBCO), 1% NEAA (GIBCO), and 0.1 mM 2-mercaptoethanol (GIBCO) before addition of the first group of reprogramming factors. For another embodiment, a basic medium may contain DMEM/F12/Glutamax (GIBCO), 10% KnockOut Serum Replacement(KSR) (GIBCO), 1% NEAA (GIBCO), and 0.1 mM 2-mercaptoethanol (GIBCO) before addition of the second group of reprogramming factors. In certain embodiments, the basic medium for the second group of reprogramming factors does not contain serum. Those skilled in the art would appreciate that further necessary supplements as described can be added to the medium.

For the culture temperature, culture at a temperature of 35.0°C or higher has been confirmed to promote cell reprogramming. The culture temperature is a temperature that does not damage the cells, such as preferably 35.0°C to 42.0°C, or more preferably 36.0°C to 40.0°C, or still more preferably 37.0°C to 39.0°C.

Reprogramming factors are molecules when contacted with a cell (e.g., expressed by a cell, transformed into a cell for expression, exogenously provided to a cell, etc.), can, either alone or in combination with other molecules, cause reprogramming. Reprogramming factors may be provided from an exogenous source, e.g., by being added to the culture media, and may be introduced into cells by methods known in the art such as through coupling to cell entry peptides, protein or nucleic acid transfection agents, lipofection, electroporation, biolistic particle delivery system (gene gun), microinjection, and the like. In certain embodiments, the reprogramming factors are added to the culture media without coupling to any other components.

In certain embodiments, the first group of reprogramming factors that can be used to reprogram the first type of cells to the second type of cells may comprise a glycogen synthases kinase 3 (GSK3) inhibitor, a transforming growth factor-β (TGFβ) inhibitor, and a cyclic AMP inducer. In other embodiments, the first group of reprogramming factors may further comprise a basic fibroblast growth factor (bFGF), a DNA methyltransferase inhibitor, a histone methyltransferase inhibitor (e.g. DOT1L inhibitor), a histone deacetylase inhibitor, BMP4 or a combination thereof.

In certain embodiments, the first group of reprogramming factors may be consisted of a GSK3 inhibitor, a TGFβ inhibitor, and a cyclic AMP inducer. In certain embodiments, the first group of reprogramming factors may be consisted of a GSK3 inhibitor, a TGFβ inhibitor, a cyclic AMP inducer and bFGF. In certain embodiments, the first group of reprogramming factors may be consisted of a GSK3 inhibitor, a TGFβ inhibitor, a cyclic AMP inducer, a DNA methyltransferase inhibitor, a histone deacetylase inhibitor, and BMP4. In certain embodiments, the first group of reprogramming factors may be consisted of a GSK3 inhibitor, a TGFβ inhibitor, a cyclic AMP inducer, a DNA methyltransferase inhibitor, a histone methyltransferase inhibitor (e.g. DOT1L inhibitor), a histone deacetylase inhibitor, and BMP4.

In certain embodiments, the reprogramming factors can sometimes be functionally replaced by paralogs within their respective families.

As used herein, the term "inhibitor" refers to an agent which can decrease the expression and/or activity of the targeted expression product (e.g. mRNA encoding the target or a target polypeptide), e.g. by at least 10% or more, e.g. by 10% or more, 50% or more, 70% or more, 80% or more, 90% or more, 95% or more, or 98% or more. The efficacy of an inhibitor, e.g. its ability to decrease the level and/or activity of the target, can be determined, e.g. by measuring the level of an expression product of and/or the activity of the target. Methods for measuring the level of a given mRNA and/or polypeptide are known to one of skill in the art, e.g. RT-PCR can be used to determine the level of RNA and Western blotting with an antibody can be used to determine the level of a polypeptide. The activity of a target can be determined using methods known in the art and described herein, e.g. transcriptional activity assays. In some embodiments, the inhibitor can be an inhibitory nucleic acid; an aptamer; an antibody or its binding fragment; or a small molecule.

GSK3 (glycogen synthase kinase 3), which is a serine/threonine protein kinase, is involved in many signal pathways relating to glycogen production, apoptosis, maintenance of stem cell and the like. GSK3 includes isoforms (GSK3α and GSK3β) encoded by different genes and having high homology at the amino acid level. Examples of the GSK3 inhibitors include GSK3α inhibitors and GSK3β inhibitors. Specific examples of the GSK3 inhibitors may include siRNAs against the gene encoding GSK3, anti-GSK3 antibodies, CHIR98014 (Milipore, Bedford), CHIR99021 (Milipore, Bedford), Kenpaullone (Milipore, Bedford), AR-AO144-18 (Santa Cruz Biotechnology, Santa Cruz), TDZD-8 (Abcam, Cambridge, US), SB216763 (Abcam, Cambridge, US), BIO ((2'Z, 3'E)-6-Bromoindirubin-3'-oxime) (R&D Systems, Minneapolis, US), TWS-119 (Abcam, Cambridge, US), SB415286 (Abcam, Cambridge, US), Ro3303544 (US6479490), LiCl, Li₂CO₃, and the like. All of these are commercially available, or can also be prepared by those of ordinary skill in the art, by reference to known documents.

In certain embodiments, the GSK3 inhibitor may be selected from a group consisting of CHIR99021, LiCl, Li₂CO₃, and BIO. In certain embodiments, the GSK3 inhibitor may be CHIR99021. In certain embodiments, the GSK3 inhibitor may be BIO. The concentration of the GSK3 inhibitor(s) in a medium may be appropriately determined according to the kind of the inhibitor to be used. In the case of CHIR99021 or BIO, the concentration may be generally 0.1-10 µM, preferably 1-5 µM, more preferably about 3 µM. One or more kinds of GSK3 inhibitors may be used in combination.

TGFβ (transforming growth factor beta) is a multifunctional cytokine belonging to the transforming growth factor superfamily. TGFβ includes three different mammalian isoforms (TGFβ 1 to 3, HGNC symbols TGFBETA1, TGFBETA2, TGFBETA3). TGFβ is secreted by many cell types, including macrophages, in a latent form in which it is complexed with two other polypeptides, latent TGFβ binding protein (LTBP) and latency-associated peptide (LAP). The derivation of TGFβ inhibitor to be used in the present invention is not particularly limited as long as it is effective for inhibiting TGFβ functions. TGFβ inhibitor(s) may be commercially available, or can also be prepared by those of ordinary skill in the art, by reference to known documents. Specific examples of the TGFβ inhibitors may include siRNAs against the gene encoding GSK3, anti-TGFβ antibodies and chemical antagonists.

In certain embodiments, the TGFβ inhibitor may be selected from a group consisting of SB431542 (Tocris Bioscience, Bristol, UK), Repsox (Tocris Bioscience, Bristol, UK), LDN193189 (Tocris Bioscience, Bristol, UK), and Tranilast (Rizaben). In certain embodiments, the TGFβ inhibitor may be SB431542. The concentration of the TGFβ inhibitor(s) in a medium may be appropriately determined according to the kind of the inhibitor to be used. In the case of SB431542, the concentration may be generally 0.1-20 µM, preferably 1-10 µM, more preferably about 5 µM. In the case of Repsox, the concentration may be generally 0.1-20 µM, preferably 1-15 µM, more preferably about 10 µM.

The term "cyclic AMP inducer", as used herein, refers to any compound that, in an effective concentration, increases the intracellular concentration of cAMP in a cell by at least 2%, preferably by at least 5%, more preferably by at least 10%, most preferably by at least 20%. Methods for measuring intracellular cAMP levels are known to those skilled in the art. Preferred cyclic AMP inducers may include isobutylmethylxanthine and forskolin. The concentration of forskolin may be generally 1-20 µM, preferably 5-15 µM, more preferably about 10 µM.

bFGF (basic fibroblast growth factor) is one kind of protein inherently present in the body, and is known to control cell growth and differentiation, and have functions such as angiogenesis, smooth muscle cell proliferation, wound therapy, tissue repair, hematopoiesis, nerve cell differentiation and the like in various tissues and organs. The derivation of bFGF to be used in the present invention is not particularly limited as long as it is effective for the reprogramming. bFGF is commercially available, or can also be prepared by those of ordinary skill in the art, by reference to known documents. For example, it can be synthesized based on known base sequences and amino acid sequences, for example, the amino acid sequences can be obtained according to NCBI accession number of AAA52448.1 (human) and AAA37621.1 (mouse). The concentration of bFGF in a medium may be generally 1-50 ng/ml, preferably about 1-20 ng/ml, more preferably about 10 ng/ml or about 20 ng/ml.

Histone acetylation is a reversible modification, with deacetylation being catalyzed by a family of enzymes termed histone deacetylases (HDACs). Inhibitors of HDACs may include valproic acid (VPA), trichostatin A (TSA), vori-nostat (suberoylanilide hydroxamic acid, SAHA, Merck & Co., Inc.), depsipeptide (Romidepsin, FK-228, Gloucester Pharmaceutical Inc.), trapoxin, depudecin, FR901228 (Fujisawa Pharmaceuticals), and butyrate. In certain embodiments, the HDAC inhibitor may be VPA. The concentration of the HDAC inhibitor(s) in a medium may be appropriately determined according to the kind of the inhibitor to be used. In the case of VPA, the concentration is generally 1-2000 µM, preferably 10-1000 µM, more preferably about 500 µM.

The basic medium supplemented with the first group of reprogramming factors are refreshed every 1, 2 or 3 days.

In certain embodiments, the first type of cells may be cultured in the presence of the first group of reprogramming factors for at least 1, 2, 3, 4, 5, 6, 7, 8, 9, 10, 11, 12, 13, 14, 15, 16,17, 18, 19, 20, 25, 30 days, 1.5 months or 2 months. In certain embodiments, the first type of cells may be cultured in the presence of the first group of reprogramming factors for no more than 2 months, 1.5 months, 30, 25, 20, 19, 18, 17, 16, 15, 14, 13, 12, 11, 10, 9, 8, 7, 6, 5, 4, 3, 2, or 1 days. In certain embodiments, the first type of cells may be cultured in the presence of the first group of reprogramming factors from 1 day to 2 months, from 1 day to 1 month, from 1 day to 25 days, from 1 day to 20 days, from 1 day to 19 days, from 1 day to 18 days, from 1 day to 17 days, from 1 day to 16 days, from 5 day to 16 days, from 7 day to 16 days, from 8 day to 16 days, from 9 day to 16 days, from 10 day to 16 days, from 11 day to 16 days, from 12 day to 16 days, from 1 day to 15 days, from 1 day to 14 days, from 1 day to 13 days, from 1 day to 12 days, from 2 day to 12 days, from 3 day to 12 days, from 4 day to 12 days, from 5 day to 12 days, from 6 day to 12 days, from 7 day to 12 days.

In one aspect, the present disclosure provides a method for reprogramming the second type of cells into a third type of cells, comprising culturing the third type of cells in the presence of second group of reprogramming factors, wherein the second group of reprogramming factors comprise a TGFβ inhibitor and a casein kinase 1 inhibitor.

Casein kinase 1 (CK1) is serine/threonine-selective enzyme that functions as regulators of signal transduction pathways in most eukaryotic cell types. CK1 isoforms are involved in Wnt signaling, circadian rhythms, nucleo-cytoplasmic shuttling of transcription factors, DNA repair, and DNA transcription. The derivation of CK1 inhibitor to be used in the present invention is not particularly limited as long as it is effective for reprogramming. CK1 inhibitors are commercially available, or can also be prepared by those of ordinary skill in the art, by reference to known documents. Specific examples of the CK1 inhibitors may include siRNAs against the gene encoding CK1, anti-CK1 antibodies and chemical antagonists. Preferably, the CK1 inhibitor may be CKI-7. The concentration of the CK1 inhibitor(s) in a medium may be appropriately determined according to the kind of the inhibitor to be used. In the case of CKI-7, the concentration may be generally 0.1-20 µM, preferably 1-10 µM, more preferably about 5 µM. Other inhibitors of the casein kinase 1 may include PF 670462 (R&D systems, Minneapolis, US), D 4476 (R&D systems, Minneapolis, US), (R)-CR8 (R&D systems, Minneapolis, US), (R)-DRF053 dihydrochloride (R&D systems, Minneapolis, US), TAK 715 (R&D systems, Minneapolis, US), PF 4800567 hydrochloride (R&D systems, Minneapolis, US), LH 846, CKI 7 dihydrochloride (R&D systems, Minneapolis, US), SR 3029 (R&D systems, Minneapolis, US), Epiblastin A (R&D systems, Minneapolis, US), PF 5006739 (R&D systems, Minneapolis, US).

In certain embodiments, the second type of cells may be ectodermal cells. In certain embodiments, the ectodermal cells may be neural crest cells (NCCs) or neural crest cell-like cells (NCC-like cells). In certain embodiments, the second type of cells may be NCCs or NCC-like cells. NCCs or NCC-like cells may be differentiated from human embryonic stem cells (hES cells), e.g., using dual SMAD inhibitors as described herein or as described in WO/2010/096496. NCCs or NCC-like cells may be differentiated from hES cells using a combination of Wnt agonists (such as e.g., Wnt3a and/or (2'Z, 3'E)-6-bromoindiaibin-3'-oxime (BIO)) and SMAD inhibitors (such as SB431542 and/or Noggin); see Menendez et al., PNAS November 29, 2011 vol. 108 no. 48 19240-19245. For example, efficient induction of NCCs or NCC-like cells was reported after contacting hES cells with SB431542 and (2'Z, 3'E)-6-bromoindiaibin-3'-oxime (BIO) (with or without Noggin), or with Wnt3a and SB431542. NCCs may also be obtainable from cultures of neural rosettes, for example by culturing hES cells on MS5 stromal feeder cells (see Lee, et al., Stem Cells 25(8), 1931-1939 (2007), which is incorporated by reference herein in its entirety). NCCs are also obtainable from numerous tissues, including in developing embryos, in the neural tube, sciatic nerve, gut, and dorsal root ganglia; and in the juvenile and adult, in the dorsal root ganglia, bone marrow, skin, heart, cornea, teeth, and caratoid body. See Nagoshi et al., Journal of Cellular Biochemistry 107: 1046-1052 (2009); Crane and Trainor, Annu. Rev. Cell Dev. Biol. 2006. 22:267-86; and Blum, Brain Research Bulletin 83 (2010) 189-193, each of which is incorporated by reference herein in its entirety. In certain embodiments, NCC-like cells are obtained by reprogramming the first type of cells according to the present application.

In some embodiments, the step of culturing the second type of cells in the presence of the second group of reprogramming factors may lead to the conversion of at least 50%, 55%, 60%, 65%, 70%,75%, 80%, 85%, 86%, 87%, 88%, 89%, 90%, 91%, 92%, 93%, 94%, 95%, 96%, 97%, 98%, 99%, or more of the cells into the third type of cells.

In certain embodiments, the third type of cells may be ectodermal cells. In certain embodiments, the third type of cells may be neural somatic cells. In certain embodiments, the third type of cells may be corneal endothelial cell-like cells (CEC-like cells).

"Corneal endothelial cells" or "CECs" refers generally to the mitochondria-rich cells that line the posterior surface of the cornea and face the anterior chamber of the eye in a living organism. In order to distinguish the chemically induced CECs (ciCECs) provided herein and primary CECs, the resulting CECs are named as corneal endothelial cell-like cells (CEC-like cells). CECs obtained through the reprogramming method disclosed herein may be identified or recognized by their exhibition of one or more of the attributes of endogenous CECs, such as expression of CEC markers, ability to form a monolayer of uniformly sized cells with a predominantly hexagonal shape, ability to form a "leaky pump" which allows leakage of solutes and nutrients from the aqueous humor to the more superficial layers of the cornea while at the same time actively pumping water in the opposite direction, from the stroma to the aqueous. Exemplary CEC markers may include, but are not limited to: Na⁺/K⁺ ATPase, tight junction protein 1 (TJPl/ZO-1), KLF13, AQP1, Collagen VIII, SLC 16A3, CFTR, NBC1, CA2, AE2/ SCL4A2, SCL16A1, CA12, CA4, FoxCl. For example, CECs typically express CollagenVIII, Na⁺K⁺ATPase pump, and ZO-1, and do not express vWF and CD31 (the latter being present in vascular endothelial cells). In addition, CECs may express one or more corneal endothelial pump markers (which include AQP1, CA2, CA4, CA12, SCL14A2, SLC 16A1 , SLC 16A3, SLC 16A7, CFTR, NHE1, ADC Y10, voltage-dependent anion channels VDAC2 and VDAC3, chloride channel proteins CLCN2 and CLC), periocular neural crest markers (which include PITX2 and FOXCl), and/or cell adhesion and matrix proteins (which include Occludin, Connexin 43, 9.3E antigen, Collagen III, Collagen IV, N cadherin, VE cadherin, E cadherin, beta catenin, Laminin alpha 4, Nidogen-2, and Netrin 4). For example, CECs may express at least one corneal endothelial pump marker, at least one periocular neural crest marker, and at least one cell adhesion and matrix protein.

In some embodiments, the resulting CEC-like cells exhibit one or more biomarkers consistent with the primary CEC phenotype. In some embodiments, the resulting CEC-like cells may express tight junction protein 1 (TJPl/ZO-1), N-Cadherin, and Na+/K+ ATPase. In some embodiments, the expression of CEC biomarkers may be increased over 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500, 600, 700, 800, 900, 1000, 1100, 1200, 1300, 1400, 1500, 1600, 1700, 1800, 1900, 2000 folds or more relative to the NCCs or NCC-like cells from which the CEC-like cells are derived.

The markers can be detected or measured by methods known in the art. For example, the adhesive junction by N-cadherin can be confirmed by examining expression at a protein level (e.g., method utilizing antigen antibody reaction) or gene level (e.g., method utilizing RT-PCR). The Na⁺/K⁺-ATPase pumping function of the cell can be measured, for example, according to the methods described in Investigative Ophthalmology & Visual Science, 2010 vol. 51, No. 8, 3935-3942, and Current Eye Research, 2009 vol. 34, 347-354 and using the Ussing chamber.

In certain embodiments, the second group of reprogramming factors may further comprise BMP4 and/or DNA methyltransferase inhibitors.

Bone morphogenetic proteins (BMPs) are a group of growth factors encouraging the formation of bone and cartilage. BMPs interact with specific receptors on the cell surface, referred to as bone morphogenetic protein receptors (BMPRs). Signal transduction through BMPRs results in mobilization of members of the SMAD family of proteins. The signaling pathways involving BMPs, BMPRs and Smads are important in the development of the heart, central nervous system, and cartilage, as well as post-natal bone development. They have an important role during embryonic development on the embryonic patterning and early skeletal formation. As such, disruption of BMP signaling can affect the body plan of the developing embryo. Examples of BMP inhibitors may include, but are not limited to, DMH2 (Milipore, Bedford, US), Dorsomorphin (Abcam, Cambridge, US), LDN193189 (Tocris Bioscience, Bristol, UK), DMH-1 (Tocris Bioscience, Bristol, UK), K 02288 (Tocris Bioscience, Bristol, UK), and ML 347(Tocris Bioscience, Bristol, UK). The concentration of the BMP inhibitor(s) in a medium may be appropriately determined according to the kind of the inhibitor to be used, such as 0.1nM-10 µM, 0.1 nM -5 µM, 0.1 nM -2.5 µM, 0.1 nM -2 µM, 0.5 nM -2 µM, 1 nM -2 µM, 1 nM -1.5 µM M, 1 nM -1000 nM, 5 nM -1000 nM, 10 nM -1000 nM, 50 nM -1000 nM, 50 nM -500 nM, 50 nM -200 nM, 100 nM -200 nM, 100 nM -150 nM, or 100 nM.

BMP4 (bone morphogenetic protein 4) is a member of the bone morphogenetic protein family which is part of the transforming growth factor-beta superfamily. BMP4 is found in early embryonic development in the ventral marginal zone and in eye, heart blood and otic vesicle. The derivation of BMP4 inhibitor to be used in the present invention is not particularly limited as long as it is effective for reprogramming. BMP4 inhibitor is commercially available, or can also be prepared by those of ordinary skill in the art, by reference to known documents. Specific examples of the BMP4 inhibitors may include siRNAs against the gene encoding BMP4, Chordin, Noggin, and anti-BMP4 antibodies. The concentration of the BMP4 inhibitor(s) in a medium may be appropriately determined according to the kind of the inhibitor to be used, such as 0.01-100 µg/ml, 0.01-50 µg/ml, 0.01-25 µg/ml, 0.01-10 µg/ml, 0.01-5 µg/ml, 0.01-1 µg/ml, 0.01-0.5 µg/ml, 0.01-0.1 µg/ml, 0.01-0.05 µg/ml, 0.05-10 µg/ml, 0.1-10 µg/ml, 0.1-5 µg/ml, 0.1-4 µg/ml, 0.1-3 µg/ml, or 0.1-2 µg/ml, or 10 ng/ml.

DNMT (DNA methyltransferase) family of enzymes can catalyze the transfer of a methyl group to DNA. DNA methylation serves a wide variety of biological functions. The derivation of DNMT inhibitor to be used in the present invention is not particularly limited as long as it is effective for reprogramming. DNMT inhibitors may be commercially available, or can also be prepared by those of ordinary skill in the art, by reference to known documents. Specific examples of the DNMT inhibitors may include siRNAs against the gene encoding DNMT, anti-DNMT antibodies and chemical antagonists.

In certain embodiments, the DNMT inhibitor may be selected from a group consisting of decitabine (Tocris Bioscience, Bristol, UK), 5-Azacytidine (Tocris Bioscience, Bristol, UK), and RG108 (Tocris Bioscience, Bristol, UK). The concentration of the DNMT inhibitor(s) in a medium may appropriately determined according to the kind of the inhibitor to be used, such as 0.1-100µM, 0.1-50µM, 0.1-25µM, 0.1-10µM, 0.5-10µM, 1-10µM, 5µM.

DOT1L (disruptor of telomeric silencing 1-like) is a group of histone methyltransferases (HMTs), which catalyzes the methylation of lysine 37 of histone H3 in chromatin. The inhibitors of DOT1L to be used in the present invention is not particularly limited as long as it is effective for reprogramming. DOT1L inhibitor(s) may be commercially available, or can also be prepared by those of ordinary skill in the art, by reference to known documents. Specific examples of the DOT1L inhibitors may include siRNAs against the gene encoding DOT1L, anti- DOT1L antibodies and chemical antagonists. In certain embodiments, the DOT1L inhibitor(s) may be small molecular DOT1L inhibitor(s). Without wishing to be bound by theory, it is known that DOT1L can use adenosylmethionine (AdoMet) as a cofactor and catalyzes the methylation of H3K27 through the AdoMet-binding site of DOT1L. Thus, any chemicals which simulates the molecular structure of AdoMet and move it from the AdoMet-binding site of DOT1L fall in the scope of the present disclosure. In certain embodiments, the DOT1L inhibitor(s) may be selected from a group of EPZ004777, EPZ5676 (also referred as pinometostat), SGC 0946 and SYC-522.

In certain embodiments, the second type of cells may be cultured in the presence of the second group of reprogramming factors for at least 1, 2, 3, 4, 5, 6, 7, 8, 9, 10, 11, 12, 13, 14, 15, 16,17, 18, 19, 20, 25, 30 days, 1.5 months or 2 months. In certain embodiments, the second type of cells may be cultured in the presence of the second group of reprogramming factors for no more than 2 months, 1.5 months, 30, 25, 20, 19, 18, 17, 16, 15, 14, 13, 12, 11, 10, 9, 8, 7, 6, 5, 4, 3, 2, or 1 days. In certain embodiments, the second type of cells may be cultured in the presence of the second group of reprogramming factors from 1 day to 2 months, from 1 day to 1 month, from 1 day to 25 days, from 1 day to 20 days, from 1 day to 19 days, from 1 day to 18 days, from 1 day to 17 days, from 1 day to 16 days, from 5 day to 16 days, from 7 day to 16 days, from 8 day to 16 days, from 9 day to 16 days, from 10 day to 16 days, from 11 day to 16 days, from 12 day to 16 days, from 1 day to 15 days, from 1 day to 14 days, from 1 day to 13 days, from 1 day to 12 days, from 2 day to 12 days, from 3 day to 12 days, from 4 day to 12 days, from 5 day to 12 days, from 6 day to 12 days, from 7 day to 12 days.

In one aspect, the present disclosure provides a method of reprogramming a first type of cells into a third type of cells, comprising the following steps: (a) culturing the first type cells in the presence of a first group of reprogramming factors, wherein the first group of reprogramming factors comprises a glycogen synthases kinase 3 (GSK3) inhibitor, a transforming growth factor (TGFP) inhibitor, and a cyclic AMP inducer; and (b) culturing cells obtained from step (a) in the presence of a second group of reprogramming factors, wherein the second group of reprogramming factors comprises a TGFβ inhibitor and a casein kinase 1 inhibitor.

In one aspect, the present disclosure provides a method of reprogramming a first type of cells into a third type of cells, comprising a step of culturing the first type of cells in the presence of a first group of reprogramming factors and a second group of reprogramming factors, wherein the first group of reprogramming factors comprises a glycogen synthases kinase 3 (GSK3) inhibitor, a transforming growth factor (TGFβ) inhibitor, and a cyclic AMP inducer, and the second group of reprogramming factors comprises TGFβ inhibitor and casein kinase 1 inhibitor.

In certain embodiments, the first group of reprogramming factors may further comprise a bFGF. In certain embodiments, the second group of reprogramming factors may further comprise BMP4 and/or a DNA methyltransferase inhibitor.

In some embodiments, the step of culturing the first type of cells in the presence of the first and second groups of reprogramming factors may lead to the conversion of at least 50%, 55%, 60%, 65%, 70%,75%, 80%, 85%, 86%, 87%, 88%, 89%, 90%, 91%, 92%, 93%, 94%, 95%, 96%, 97%, 98%, 99%, or more of the cells into the third type of cells.

In certain embodiments, the method may further comprise a step of washing cells obtained from step (a), before starting step (b). In certain embodiments, there is no washing step between steps (a) and (b).

### C. Pharmaceutical Compositions and Therapeutic Methods

In one aspect, the present disclosure provides a population of NCC-like cells produced according to the method provided herein. In certain embodiments, at least 50%, at least 60%, at least 70%, at least 80%, or at least 90% of the population are NCC-like cells.

In one aspect, the present disclosure provides a method for producing patient-specific neural crest cell-like cells. In one embodiment, the first type of cells may be obtained from a subject suffering from a neurological disease.

In one aspect, the present disclosure provides a population of corneal endothelial-like cells (CEC-like cells) produced according to the method provided herein. In certain embodiments, at least 50%, at least 60%, at least 70%, at least 80%, or at least 90% of the cells in the population of the present disclosure are CEC-like cells.

In some embodiments, the NCC-like cells or CEC-like cells produced according to the method provided herein may be purified *ex vivo.* In some embodiments, the methods for reprogramming may yield NCCs-like cells or CEC-like cells with high purity and no purification is needed. For example, in some embodiments, the reprogramming methods provided herein may result in a cellular composition comprising at least 50%, 60%, 70%, 80%, 90%, 95%, 98%, 99%, or more NCC-like cells or CEC-like cells. In some embodiments, the reprogramming methods provided herein may yield NCC-like cells or CEC-like cells with low purity or less-than-desired purity, and purification is desired. In some embodiments, the NCC-like cells or CEC-like cells may be purified by substantially separating the NCC-like cells or CEC-like cells from other cells in the composition. In the situation of CEC-like cells purification, the other cells in the composition may include undifferentiated NCC-like cells and/or NCC-like cells that differentiated into an undesired cell lineage or phenotype.

In one aspect, the present disclosure provides a composition comprising the NCC-like cells or CEC-like cells produced according to the method provided herein. The composition may include one or more pharmaceutically acceptable carriers and diluents.

The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof. Pharmaceutical acceptable carriers for use in the pharmaceutical compositions disclosed herein may include, for example, pharmaceutically acceptable liquid, gel, or solid carriers, aqueous vehicles, nonaqueous vehicles, antimicrobial agents, isotonic agents, buffers, antioxidants, anesthetics, suspending/dispending agents, sequestering or chelating agents, diluents, adjuvants, excipients, or non-toxic auxiliary substances, other components known in the art, or various combinations thereof.

The composition described herein may also include components that facilitate engraftment. The composition described herein may be pyrogen-free or essentially pyrogen-free, and be pathogen-free, wherein the pathogen comprises bacterial contaminants, mycoplasmal contaminants, and viruses.

The composition comprising the NCC-like cells or CEC-like cells described herein may further comprise an immunosuppressive agent or immune tolerizing agent.

In a further aspect, this disclosure is directed to therapeutic use of the CEC-like cells provided herein. For example, the instant CECs may be used in cell therapy as a graft for the treatment of a disease requiring transplantation of corneal endothelium, for example, bullous keratopathy, corneal edema, corneal leukoma and the like.

In one aspect, the present disclosure provides a method for treating a disease or condition associated with dysfunctional or injured corneal endothelial cells, comprising administrating an effective amount of the CEC-like cells or the composition comprising the CEC-like cells provided herein to a subject in need thereof.

In some embodiments, the administration of corneal endothelial cells may induce an ocular healing process. In some embodiments, the administration of corneal endothelial cells may replenishe diseased tissues. In some embodiments, the administration of corneal endothelial cells may have a regenerative effect on damaged or diseased ocular tissue.

The administration routes may include any suitable means, including, but not limited to, topical application to an ocular site, injection into an ocular site, grafting at an ocular site, and the like. In some embodiments, the particular mode of administration selected will depend upon the particular treatment, disease state or condition of the patient, the nature or administration route of other drugs or therapeutics administered to the subject, etc. In some embodiments, the corneal endothelial cells may be administered to a subject in a single dose or in multiple doses over selected time intervals, e.g., to titrate the dose. When multiple doses are administered, the doses may be separated from one another by, for example, one week, one month, one year, or ten years. One or more growth factors, hormones, interleukins, cytokines, small molecules or other cells may also be administered before, during, or after administration of the cells to further bias them towards a particular cell type.

The term "effective amount", as used herein, refers broadly to the amount of a compound or cells that, when administered to a patient for treating a disease, is sufficient to effect such treatment for the disease. The effective amount may be an amount effective for prophylaxis, and/or an amount effective for prevention. The effective amount may be an amount effective to reduce, an amount effective to prevent the incidence of signs/symptoms, to reduce the severity of the incidence of signs/symptoms, to eliminate the incidence of signs/symptoms, to slow the development of the incidence of signs/symptoms, to prevent the development of the incidence of signs/symptoms, and/or effect prophylaxis of the incidence of signs/symptoms. The "effective amount" may vary depending on the disease and its severity and the age, weight, medical history, susceptibility, and preexisting conditions, of the patient to be treated. The term "effective amount" is synonymous with "therapeutically effective amount" for purposes of this invention.

"Treating" or "treatment", as used herein, covers the treatment of a disease or medical condition described herein, in a subject, such as a human, an animal, or a mammal, and may include: (i) inhibiting a disease or disorder, i.e., arresting its development; (ii) relieving a disease or disorder, i.e., causing regression of the disorder; (iii) slowing progression of the disorder; and/or (iv) inhibiting, relieving, or slowing progression of one or more symptoms of the disease or medical condition.

As used herein, the term "subject" is not limited to a specific species or sample type. For example, the term "subject" may refer to a patient, and frequently a human patient. However, this term is not limited to humans and thus encompasses a variety of mammalian species, such as non-human veterinarian mammal, such as dogs, cats, rabbits, pigs, rodents, horses, or monkeys.

The CEC-like cells provided herein may be a cell mass such as pellet and its analogue obtained by concentration and filtration, and the analogue can be used as the medicament of the present invention. Moreover, it is also possible to add a protector such as glycerol, DMSO (dimethyl sulfoxide), propylene glycol, acetamide and the like to the medicament, and cryopreserve the mixture. For safer utilization of a medicament, the medicament may be subjected to a treatment under the conditions causing denaturation of pathological proteins, such as a heat treatment, a radiation treatment and the like, while retaining the function of the corneal endothelial cells.

In some embodiments, the CEC-like cells can be administrated in combination with surgery. In some embodiments, the surgery may be Descemet's stripping with endothelial keratoplasty (DSEK), which includes the removal of Descemet's membrane and the corneal endothelium, and subsequent transplantation of a donor tissue. Alternatively, the surgery can be penetrating keratoplasty (PKP), in which the entire cornea is removed and replaced. Other surgery may include lamellar keratoplasty, Descemet's Membrane Endothelial Keratoplasty (DMEK), DSAEK, and DLEK.

The "disease or condition associated with dysfunctional or injured corneal endothelial cells" may include any disease or condition suitable to be treated by the administration of CEC-like cells, including diseases in which a subject's CECs decrease in numbers or die, decrease in density, or otherwise become dysfunctional. Primary diseases that affect the corneal endothelium may include Fuch's dystrophy, iridocorneal endothelial syndrome, posterior polymorphous dystrophy, and congenital hereditary endothelial dystrophy. Secondary diseases or conditions for which an effective treatment may include replacement of the corneal endothelium, may include age-related macular degeneration (AMD), retinitis pigmentosa, glaucoma, corneal dystrophies, contact lens usage, cataract surgery, and late endothelial failure in cornea transplantation. Diseases of corneal endothelial cells may additionally include any injury to the cornea, e.g., caused by chemical irritation, injury due to contact lens use, reaction or sensitivity (e.g., to contact lens solutions, cosmetics, eye drops, medications, smoke, etc.), scratches, scrapes, abrasions, bruising, a foreign object in the eye (e.g., sand or dust), or exposure to ultraviolet light (from e.g., sunlight, sun lamps, snow reflections, water reflections, or arc-welding or other exposure). In certain embodiments, the disease or condition associated with dysfunctional or injured corneal endothelial cells may cause vision loss in the subject. In certain embodiments, the vision loss in the subject may be permanent or irreversible.

In certain embodiments, the corneal endothelial cells may be immunologically compatible with the subject (e.g. allogenic or autologous).

In certain embodiments, the method of treatment may further comprise administering an immunosuppressive agent or immune tolerizing agent to said subject. The immunosuppressive agent or immune tolerizing agent may be administered in an amount sufficient to reduce the risk of rejection of said CEC-like cells.

The immunosuppressive or immune tolerizing agent may comprise one or more of anti-lymphocyte globulin (ALG) polyclonal antibody, anti- thymocyte globulin (ATG) polyclonal antibody, azathioprine, BASILIXIMAB^{®} (anti-lL- 2Ra receptor antibody), cyclosporin (cyclosporin A), DACLIZUMAB^{®} (anti-IL-2Ra receptor antibody), everolimus, mycophenolic acid, RITUXIMAB^{®} (anti-CD20 antibody), sirolimus, tacrolimus, mycophemolate mofetil, and corticosteroids.

The immunosuppressants may be dosed at least about 1, 2, 4, 5, 6, 7, 8, 9, or 1 0 mg/kg. When the immunosuppressants are used, they may be administered systemically or locally, and they may be administered prior to, concomitantly with, or subsequent to administration of the CEC-like cells. Immunosuppressive therapy may continue for weeks, months, years, or indefinitely following administration of cells. For example, the patient may be administered 5 mg/kg cyclosporin for 6 weeks following the administration of the CEC-like cells.

In one aspect, the present disclosure provides use of the CEC-like cells or the composition provided herein in the manufacture of a medicament for treatment of a disease or condition associated with dysfunctional or injured corneal endothelial cells.

In one aspect, the present disclosure provides a method for treating a disease or condition associated with dysfunctional or injured corneal endothelial cells, comprising administrating an effective amount of the NCC-like cells or the composition comprising the NCC-like cells provided herein to a subject in need thereof.

In one aspect, the present disclosure provides use of the NCC-like cells or the composition provided herein in the manufacture of a medicament for treatment of a disease or condition associated with dysfunctional or injured corneal endothelial cells.

The NCC-like cells provided herein can be used to treat a neurological disease.

"Neurological disease" as used herein is defined as a disorder of the nervous system, and include disorders that involve the central nervous system (brain, brainstem and cerebellum), the peripheral nervous system (including cranial nerves), and the autonomic nervous system (parts of which are located in both central and peripheral nervous system). In particular neurological disease may include any disease wherein the function of neural crest cells or Schwann cells is impaired, altered or disrupted. Examples of neurological diseases connected with Schwann cells may comprise Demyelinating diseases, Multiple sclerosis, Myelopathies, Experimental allergic encephalomyelitis (EAE), acute disseminated encephalomyelitis (ADEM), postinfectious or postvaccinal encephalomyelitis, peripheral neuropathies, Schwannomatosis, Charcot-Marie- Tooth disease, Guillain-Barre Syndrome, Chronic inflammatory demyelinating polyradiculoneuropathy (CIDP).

The present disclosure also provides a method for drug discovery and/or drug screening. Assays for drug discovery and/or drug screening are also provided. In certain embodiments, the methods may include administering a drug candidate to the NCC-like cells or CEC-like cells, and detecting the response of the cells to the drug candidate. Detection of the response can identify whether the drug candidate has suitable properties (e.g., toxicity, or therapeutically effective). In some embodiments, the method can be used to determine cell health and viability in the presence of a drug candidate. In some embodiments, the method can be used to test the toxicity of a drug candidate. In some embodiments, the method can be used to assess changes in the phenotype of a cell population in the presence of a drug candidate.

In one aspect, the present disclosure provides a method of using the NCC-like cells for screening for drugs that reverse, inhibit or prevent neurological diseases, or neural side effects of medicaments, for example diabetes medicaments.

### D. Kits

In one aspect, the present disclosure provides a kit for reprograming a first type of cells into a second type of cells, wherein the kit comprises a first group of reprogramming factors, and the first group of reprogramming factors comprises a glycogen synthases kinase 3 (GSK3) inhibitor, a transforming growth factor (TGFβ) inhibitor, and a cyclic AMP inducer.

In one aspect, the present disclosure provides a kit for reprograming a second type of cells into a third type of cells, wherein the kit comprises a second group of reprogramming factors and the second group of reprogramming factors comprises a TGFβ inhibitor and a casein kinase 1 inhibitor.

In one aspect, the present disclosure provides a kit for reprograming a first type of cells into a third type of cells, wherein the kit comprises a first group of reprogramming factors and a second group of reprogramming factors, wherein the first group of reprogramming factors comprises a glycogen synthases kinase 3 (GSK3) inhibitor, a TGFβ inhibitor, and a cyclic AMP inducer, and the second group of reprogramming factors comprise a TGFβ inhibitor and a casein kinase 1 inhibitor.

The kit may further comprise an instruction of use, and a package that separates each of the components in the kit.

All publications and patents cited in this specification are herein incorporated by reference to their entirety.

### EXAMPLES

### EXAMPLE 1: MATERIAL AND METHODS

### 1. Animals

All animal experiments were approved by the Animal Ethics Committee of Wenzhou Medical University, Wenzhou, China. Oct4-GFP transgenic allele-carrying mice (CBA/CaJ×C57BL/6J) were from the Jackson Laboratory; Wnt1-cre and Fsp1-Cre mice were from the Jackson Laboratory (BALB/c-Tg (S100a4-cre)1Egn/YunkJ); ROSA26-tdTomato mice were from the Jackson Laboratory (Gt(ROSA)26Sortm14(CAG-tdTomato)Hze). 129Sv/Jae and C57BL/6 mice were from Beijing Vital River Laboratory. The Wnt1-Cre/ROSA26^{tdTomato} and Fsp1-Cre/ROSA26^{tdTomato} mice were obtained by crossing Wnt1-Cre and Fsp1-Cre mice with ROSA26-tdTomato mice, respectively (see Figure 4A). The New Zealand white rabbits were from JOINN Laboratories (Suzhou) Inc., Suzhou, China. All animals were treated in accordance to the ARVO (Association for Research in Vision and Ophthalmology) Statement for the Use of Animals in Ophthalmic and Vision Research. All animals were housed under a stable environment (21±2°C) with a 12-h dark/light cycle.

### 2. Cell Culture

Primary mouse embryonic fibroblasts (MEFs) were isolated from embryonic day 13.5 (E13.5) mouse embryos as previously described (Liu, C., Hu, X., Li, Y., Lu, W., Li, W., Cao, N., Zhu, S., Cheng, J., Ding, S., and Zhang, M. (2019). Conversion of mouse fibroblasts into oligodendrocyte progenitor-like cells through a chemical approach. Journal of molecular cell biology.; Wang, H., Cao, N., Spencer, C.I., Nie, B., Ma, T., Xu, T., Zhang, Y., Wang, X., Srivastava, D., and Ding, S. (2014). Small molecules enable cardiac reprogramming of mouse fibroblasts with a single factor, Oct4. Cell reports 6, 951-960.). Briefly, the head, tail, limbs and visceral tissues of the embryos were carefully removed and discarded. The remaining tissues were sliced into small pieces, trypsinized by 0.25% Trypsin/EDTA (Gibco) and plated onto 10-cm dish. Fibroblasts were cultured in fibroblast medium containing DMEM (Gibco) supplemented with 10% fetal bovine serum (FBS, Gibco), 2mM GlutaMAX (Gibco), 0.1mM non-essential amino acids (Sigma), 100 units/mL penicillin, and 100 mg/mL streptomycin (Gibco). All fibroblasts were expanded for two passages and then used for further experiments. To prepare the Wnt1 ⁻ MEFs, the fibroblasts derived from the Wnt1-Cre/ROSA26^{tdTomato} mouse embryos were sorted for tdTomato⁻ cells by FACS.

Mouse primary corneal endothelial cells (CECs) (CP-M179) and medium (CM-M179) were purchased from Procell Life Science & Technology Co., Ltd (Wuhan, China). Mouse primary CECs were cultured in DMEM containing 10% FBS (GIBCO), 0.1 mM non-essential amino acids (Sigma), 2mM GlutaMAX (GIBCO) and 1% penicillin streptomycin (GIBCO). mESCs were maintained in ESC medium consisting of DMEM comprising 10% FBS (GIBCO), LIF, 0.1 mM non-essential amino acids (Sigma), 2 mM GlutaMAX (GIBCO), 1% penicillin streptomycin (GIBCO), 0.1mM 2-mercaptoethanol (GIBCO), CHIR99021 (3mM) and PD0325901 (1mM).

Human embryonic fibroblasts (HEFs), human neonatal fibroblasts (HNFs) and human adult fibroblasts (HAFs) were purchased from ScienCell Research Laboratories. Human umbilical cord mesenchymal stem cells (MSCs) were kindly provided by Nuwacell Ltd. (Hefei, China). They were maintained in the fibroblast medium containing DMEM (GIBCO) supplemented with 10% FBS (GIBCO), 1% GlutaMAX (GIBCO) and 1% NEAA (GIBCO). Human exfoliated renal epithelial cells (UCs) were derived from 100-300 mL urine samples from normal people as previously reported Zhou, T. et al. (2012). Generation of human induced pluripotent stem cells from urine samples. Nature protocols 7, 2080-2089).

### 3. FACS sorting to derive the tdMEFs

To prepare the tdMEFs, the resulting fibroblasts were sorted for tdTomato⁺ /p75 cells by FACS as previously described. The primary MEFs were isolated from E13.5 mouse embryos with genetic background of Fsp1-Cre/Rosa26^{tdTomato} (Fsp1-Cre mice × Rosa26^{tdTomato} mice). The MEFs at passage 2 were dissociated with 0.25 % trypsin at 37 °C for 5 min, and neutralized with MEF medium. Those MEFs were stained with specific antibody against p75, and subjected to FACS sorting for the tdTomato ⁺/p75⁻ cells. During FACS sorting, pre-warm the Matrigel-coated 24-well plate at 37 °C for at least 30 min before seeding the tdMEFs. The tdMEFs were seeded immediately after FACS sorting into pre-warmed Matrigel-coated 24-well plates at 15,000 cells per well in MEF medium supplemented with 1 µM Thiazovivin (Tzv) in 5 % CO₂ and 20 % O₂ at 37 °C for 5 hours to allow MEFs to attach the plate. After 5 hours, medium was changed to MEF medium without Tzv, and the tdMEFs were cultured at 37 °C in 5 % CO₂ overnight.

### 4. Small-molecule compounds and libraries

Small molecules are from Sigma, including GSK3b inhibitor CHIR99021 (SML1046), TGFb inhibitor SB431542 (S4317), DNA methylation inhibitor 5-Aza-dC (A3656), the cyclic AMP inducer forskolin (F6886) and CKI-7 (C0742). bFGF is from Peprotech. DOT1L inhibitor EPZ004777 (S7353) and ROCK inhibitor Y-27632 (S1049) are obtained from Selleck.

### 5. Immunocytochemistry

To further investigate the expression of typical neural crest (NC) cell markers, the reprogrammed MEFs were fixed, immuno-stained, and analyzed. Briefly, Cells were washed once with 1×PBS and fixed with 4% paraformaldehyde at room temperature for 10 min, followed by permeabilizing with 0.2% Triton X-100 in 1×PBS for 10 min, and blocking with 7.5 % BSA for at least 1 h. All primary antibodies were diluted in 7.5 % BSA, and the incubation was performed at 4 °C overnight. The cells were washed with 1×PBS 10 min for five times at room temperature. The secondary antibodies, Alexa-488, Alexa-555, and Alexa-647, purchased from Invitrogen, were diluted into 7.5 % BSA, and the incubation was lasted for 1 h at room temperature, followed by five 10 min-washes with 1×PBS. The nuclei were stained with DAPI. Antibodies used in this study were listed in Table 1.

**Table 1: Antibodies used in this study**

| **Antigen** | **Antibody Type** | **Company** | **Cat No** |
|---|---|---|---|
| anti-actin-α-smooth | Mouse | Sigma | A5228 |
| AP2α | Rabbit | Abcam | ab108311 |
| AQP1 | Rabbit | Abcam | ab168387 |
| Na⁺/K⁺ -ATPase A | Mouse | Abcam | ab2826 |
| Na⁺/K⁺ -ATPase | Mouse | Thermo Fisher Scientific | MA3-912 |
| Bestrophin | Rabbit | Abcam | ab14927 |
| CD105 | Mouse | eBioscience | 12-1051-82 |
| CD90 | Mouse | eBioscience | 12-0900-81 |
| CD73 | Mouse | eBioscience | 12-0731-81 |
| CRALBP | Mouse | Abcam | ab15051 |
| FoxC1 | Goat | Abcam | ab5079 |
| GFAP | Rabbit | Sigma | HPA056030-100UL |
| HNk1(CD57) | Rabbit | Abcam | ab221756 |
| laminin | Rabbit | Abcam | ab11575 |
| Mitf | Mouse | Exalpha | X1405M |
| Nanog | Rabbit | Abcam | ab109250 |
| Nestin | Rabbit | BOSTER | BA1289 |
| Nestin | Mouse | Abcam | ab11306 |
| Oct4 | Rabbit | Abcam | ab18976 |
| Otx2 | Rabbit | Abcam | ab183951 |
| P75 NGF Receptor | Rabbit | Abcam | ab52987 |
| PAX6 | Rabbit | Abcam | ab195045 |
| Peripherin | Mouse | Santacruz | sc-377093 |
| PITX2 | Rabbit | Abcam | ab 192495 |
| Retinal RX | Mouse | Santacruz | sc-271889 |
| S100 β | Rabbit | Abcam | ab52642 |
| SLUG | Mouse | Abcam | Ab51772 |
| Sox1 | Rabbit | Abcam | ab109290 |
| Sox10 | Rabbit | Abcam | ab155279 |
| Sox2 | Mouse | Santacruz | sc-365823 |
| Sox9 | Rabbit | Abcam | ab 185966 |
| SSEA-3 | Rabbit | Millipore | FCMAB141A4 |
| SSEA-3 | Rat | Millipore | 2739108 |
| SSEA-4 | Mouse | Thermofisher | MC-813-70 |
| ZO-1 | Rabbit | Abcam | ab216880 |
| Goat IgG (H+L) secondary antibody | Donkey | Thermo Fisher Scientific | A32814 |
| Mouse IgG (H+L) secondary antibody | Goat | Thermo Fisher Scientific | A32723 |
| Alexa Fluor594 AffiniPure Donkey AntiMouse IgG (H+L) | / | Jackson ImmunoResearch | 715-585-151 |
| Donkey anti-goat IgG (H+L) secondary antibody, Alexa Fluor 555 conjugate | / | Thermo Fisher Scientific | A-21432 |
| RNeasy Mini Kit (50) | / | QIAGEN | 74104 |
| RNA 6000 analytical Nano Kit | / | Agilent Technologies | 5067-1511 |
| HiSeq PE Cluster Kit V4-cBot-HS | / | Illumina | PE-401-4001 |

### 6. Statistical analysis

All experiments were independently performed at least three times. The results are expressed as means ± SD. The data were analyzed by unpaired two-tailed Student's t tests for comparisons of two groups and by one-way analysis of variance (ANOVA) with Tukey's test or Dunnett's multiple comparisons test for comparisons of multiple groups. All analyses were performed using SPSS Statistics 19.0 software. P < 0.05 was considered significant.

### 7. Cell cycle analysis

Cells were carefully dissociated into single-cell suspensions by Accutase, washed twice with PBS, and then fixed overnight with cold 70% ethanol. Fixed cells were washed twice with PBS, followed by ribonuclease (100 µg/ml; Sigma-Aldrich) treatment and propidium iodide (PI) (50 µg/ml; Sigma-Aldrich) staining for 30 min at 37°C. Approximately 1×10⁶ cells were analyzed using FACSCanto II (Becton Dickinson) to determine the cell cycle distribution pattern. The percentages of cells in the G1, S, and G2/M phases of the cell cycle were analyzed using ModFit 4.1 (Verity Software House).

### 8. Transplantation

All rabbits weighing 2.0 to 2.5 kg were anesthetized intramuscularly with ketamine hydrochloride (60 mg/kg) and xylazine (10 mg/kg; Bayer, Munich, Germany). The rabbits were divided into two groups (n = 10 each group), and the right eye was used for this experiment. After disinfection and sterile draping of the operation site, a 6-mm corneal incision centered at 12 o'clock was made with a slit knife, and a viscoelastic agent (Healon; Amersham Pharmacia Biotech AB) was infused into the anterior chamber. After the corneal surface had been ruled with a marking pen (Devon Industries Inc., Madrid, Spain), a 6.0-mm-diameter circular aperture for descemetorhexis was created in the center of the cornea with a 30-gauge needle (Terumo, Tokyo, Japan), and Descemet's membrane was removed from the anterior chamber of the eye. The corneal endothelium was mechanically scraped from Descemet's membrane with a lacrimal passage irrigator (Shandong Weigao) as previously described. Fsp-ciCECs were dissociated using 0.25% trypsin-EDTA, resuspended in basic medium at a density of 1×10⁷ cells/ml, and kept on ice. The anterior chamber was washed with PBS three times. After this procedure, a 26-gauge needle was used to inject 1×10⁶ cultured ciCECs suspended in 100 µl of basic DMEM containing 10 µM ROCK inhibitor Y-27632 (Selleck) into the anterior chamber of the right eye. Thereafter, rabbits in the frozen group (solely with cryodamage), CE transplantation eye-down group (cryodamage and CE injection) and sphere eye-down group (cryodamage and sphere injection) were kept in the eye-down position for 24 hours under deep anesthesia so the cells could become attached by gravitation. Rabbits in the sphere eye-up group were kept in the eye-up position for 24 hours under deep anesthesia. Each surgical eye was checked two or three times a week by external examination, and photographs were taken on days 3, 7, 14, and 28 after injection. Central corneal thickness was measured using ultrasound pachymeter, and intra-ocular pressure was measured with pneumatic tonometer on days 0.5, 1, 3, 7, 14, 21 and 28 after surgery. An average of three readings was taken.

### 9. Cell proliferation assay

The proliferation rate of ciCECs cultured in an individual differentiation medium or an individual M5 was determined by the Click-iT^{™} 5-ethynyl-2'-deoxyuridine (EdU) Alexa Fluor 488 Imaging Kit (Qiagen) according to the manufacturer's instructions. Briefly, passaged CECs were seeded onto a slide at a lower density of 5×10³ cells per cm² and cultured for 24 hours.

### 10. Transmission electron microscopy (TEM) analysis

For TEM analysis, cells were fixed in 2.5% EM-grade glutaraldehyde (Servicebio) for 2 to 4 hours at 4°C, washed with 0.1 M phosphate buffer (pH 7.4), postfixed in 1% osmium tetroxide for 2 to 4 hours at 4°C, and washed and then dehydrated in an ethanol series (50 to 100%) to a final rinse in 100% acetone, followed by 2-hour incubations in 1:1 acetone/Pon 812 (SPI) and overnight incubation in 1:2 acetone/Pon 812. The samples were embedded in Pon 812, polymerized for 48 hours at 60°C, and then sectioned (60 to 80 nm) with a diamond knife (Daitome). Sections were stained with 2% uranyl acetate, followed by lead citrate, and visualized using an HT7700 transmission electron microscope (HITACHI).

### 11. Karyotype analysis

Cells were treated with colcemid (0.1 µg/ml) (Gibco) at 37°C for 2 hours, trypsinized, resuspended, and incubated in 0.075 M potassium chloride for 15 min at 37°C, fixed with 3:1 methanol:acetic acid, and then dropped onto slides to spread the chromosomes. The chromosomes were visualized by Giemsa (Solarbio) staining.

### 12. RNA sequencing and analysis

Total RNA for each sample was isolated with TRIzol reagent and purified using the RNeasy 23 Mini Kit (Qiagen) according to the manufacturer's instructions. RNA quality and quantity were assessed using NanoDrop 2000, Agilent 2100 Bioanalyzer, and Agilent RNA 6000 Nano Kit. RNA library construction and RNA sequencing were performed by the Annoroad Gene Technology. Sequencing libraries were generated using the NEB Next Ultra RNA Library Prep Kit for Illumina24 (NEB), and library clustering was performed using HiSeq PE Cluster Kit v4-cBot-HS (Illumina) following the manufacturer's recommendations. After cluster generation, the libraries were sequenced on an Illumina platform and 150-base pair pairedend reads were generated. The initial data analysis was performed on BMKCloud (www.biocloud.net/).

### 13. FACS cytometry

For MEF preparation, fibroblasts with the desired genotype were cultured in MEF medium until they reached more than 80% confluence. The cells were washed twice with 1× PBS and treated with 0.25% trypsin at 37°C for 5 min. After harvesting, the cells were passed through a 70-µm filter, washed twice with a precooled buffer (1×PBS, 1.5% FBS, and 0.5% BSA), and resuspended in said buffer. The cells were incubated with either fluorescein isothiocyanate (FITC)-conjugated P75 antibody (Abcam) or isotype control (BD) at the suggested concentrations on ice for 30 min or room temperature for 45 min, followed by six washes with FACS buffer. Cells were then resuspended in FACS buffer and sorted with BD FACSAria II.

### EXAMPLE 2: GENERATION OF CINCCS AND CICECS FROM FIBROBLASTS

### 1. Culture medium preparation

### Stage I medium preparation

The basal medium which contains DMEM/F12/Glutamax (GIBCO), 10% KSR (GIBCO), 10% FBS(GIBCO), 1% NEAA (GIBCO), 0.1 mM 2-mercaptoethanol (GIBCO) was supplemented with small molecules RepSox (10 µM), Chir99021 (10 µM), Forskolin (10 µM), and bFGF (10 ng/ml).

Before adding the first set of reprogramming factors, the basal medium can further comprise DMEM/F12/Glutamax (GIBCO), 0.075% bovine serum albumin (BSA) (GIBCO), 1% NEAA (GIBCO), and 0.1 mM 2-mercaptoethanol (GIBCO) and can provide comparable or better effect in cell reprogramming (data not shown).

In addition, the combination of small molecules RepSox (10 µM), Chir99021 (10 µM), and Forskolin (10 µM) can achieve similar induction effects (data not shown). The medium was shaked for 30 min to ensure fully dissolution.

### Stage II medium preparation

DMEM/F12/Glutamax (GIBCO), 10% KSR (GIBCO), 1% NEAA (GIBCO), 0.1 mM 2-mercaptoethanol (GIBCO), supplemented with 5 µM SB431542 and 5 µM CKI-7.

### 2. Chemical induction of ciCECs from fibroblasts

To investigate whether small molecules can chemically reprogram fibroblasts into corneal endothelium cells, we devised a two-step process to directly reprogram mouse fibroblasts into neural crest cells and differentiation into corneal endothelial cells (Figures 1A). In the Wnt1-Cre-Rosa^{Tomato} mice, tdTomato is faithfully expressed in the neural crests (NCs) under control of the Wnt1 gene. Therefore, MEFs were isolated from Wnt1-Cre/ROSA26^{tdTomato} mouse at embryonic stage E13.5, and the neural crest population was marked with tdTomato expression. We performed fluorescence-activated cell sorting (FACS) to collect the tdTomato⁻ population to exclude any neural crest or progenitors. These MEFs were negative for typical NC markers, including Sox10, P75, Hnk1 and AP2α (data not shown). In addition, these MEFs were also negative for typical neural stem cell (NSC) markers, including Sox2, Pax6, and Olig2 (data not shown). The method for inducing NC from fibroblasts by small molecules has not been reported. To investigate whether small molecules can chemically reprogram fibroblasts into ciNCCs, we selected more than 20 small molecules as our potential candidate molecules for neural crest lineage reprogramming based on that target epigenetic modifications and modulate neural crest-developmental signaling. The candidate small molecules were focused on three primary categories of reprogramming factors comprised of three small molecules: (1) TGF-β signaling inhibitors such as RepSox (R), which inhibit mesoderm and endoderm specification; (2) GSK3 inhibitors such as CHIR99021 (C), which favor neural development and (3) cyclic AMP inducers such as Forskolin (F). These three compounds were combined with basic fibroblasts growth factor (bFGF) as chemically defined medium (hereafter termed RCF) to reprogram mouse embryonic fibroblasts (MEFs) into ciNCCs.

Briefly, MEFs were plated at 50,000 cells per well in a 6-well plate. After overnight culture, MEFs were treated in RCF chemically defined reprogramming medium. In the RCF medium, many small compact cell clusters with clear-cut edges quickly appeared within twelve days (Figure 1B). To identify the combination of RCF sufficient for reprogramming mouse fibroblasts into chemical-induced neural cell-like cells (ciNCCs), Wnt1 MEFs were used to observe the tdTomato expression. The result showed that the RCF compounds can reprogram MEFs into tdTomato positive neural crest-like cells (Figure 1D). To further investigate the expression of typical NC markers, the RCF-treated MEFs were fixed, immuno-stained, and analyzed. These cells expressed HNK1, P75 and AP2α (Figure 1F). Those fibroblast-originated, highly proliferative, and self-renewable Sox10-positive cells are thereafter referred to as ciNCCs.

During the reprogramming process, many small compact cell clusters with clear-cut edges quickly appeared within 7 days (Figure 1B and Figure 1C). After RepSox, Chir99021, Forskolin and bFGF treatment, we observed tdTomato⁺ cells after 10-day treatment (Figure 1D and Figure 1E). The markers for neural crest cells (NCCs) (HNK1, P75 and AP2α) on day 12 were observed (see Figure 1F). However, the combination of small molecules RepSox (10 µM), Chir99021 (10 µM), and Forskolin (10 µM) without bFGF can achieve similar induction effects (data not shown).

After inducing ciNCCs with a differentiation medium with SB431542 and CKI-7 for 14 days, the ciNCCs were differentiated into corneal endothelial-like cells (ciCECs) (see Figure 3C). Figure 3A showed the markers of ciCECs at day 14 of Stage II induction detected by immunofluorescence staining (Na⁺-K⁺ ATPase, AQP1, Vimentin, N-cadherin, laminin and AQP1). The tight junction of corneal endothelial cells were also observed under the Transmission electron microscope.

To avoid possible contamination of neural crest cells (NCCs) in the starting MEFs, we carried out a lineage-tracing experiment to track the origin of the ciNCCs and ciCECs (Figure 4B). In the Fsp1-Cre-RosaTomato mice, tdTomato is faithfully expressed in the ciNCCs and ciCECs under control of the Fsp1 gene. The expression of the NC markers P75, Hnk1, AP2α, and SOX10 were observed by immunocytochemistry in the Fsp1-ciNCCs (see Figure 4C). Figure 4D showed the representative morphology changes of Fsp1-Cre:R26RtdTomato MEFs and ciCEC induced from these MEFs.

Human cells (human embryonic skin fibroblasts (HEF), human neonatal fibroblasts (HNF), human adult fibroblasts (HAF), human umbilical cord mesenchymal stromal cells (MSCs) and urine cells (UC) were induced by the chemical factors using the same procedure as described above. Preferably, the chemical factors added in Stage I are RepSox (10 µM), Chir99021 (10 µM), Forskolin (10 µM), 5-Azacytidin (5 µM), VPA (500 µM) and BMP4 (10 ng/ml). The chemical factors added in Stage II are 5 µM SB431542 and 5 µM CKI-7, identical as mentioned above. The induced ciNCCs and ciCECs derived from the above cells are shown in Figure 5.

### EXAMPLE 3: CHARACTERIZATION OF CINCCS AND CICECS OBTAINED FROM EXAMPLE 2

### 1.1 RNA preparation and RT-PCR

In order to confirm the expression of NC genes, including Sox10, P75, Pax3, and Msx1. Total RNA was extracted using RNeasy Plus mini kit (Qiagen). In brief, 1 µg total RNA was used for reverse transcription reaction with iScript cDNA synthesis kit (Bio-Rad), and the resulting cDNA was diluted five times in H₂O for PCR. For the semi-quantitative PCR, 1 µl of 1/5 diluted cDNA was used as template for PCR program: 95°C 5 min, and 35 cycles of 95°C 30 s, 60°C 30 s, and 72°C 30 s, followed by 72°C 10 min. Quantitative PCR was performed following the protocol of FAST SYBR Green Master Mix (ABI). All PCR was performed in triplicate, and the expression of individual genes was normalized to that of *Gapdh.* Primer sequences are listed in Table 2.

**Table 2: Primers for qRT PCR**

| **Gene** | **Forward sequence** | **Reverse sequence** |
|---|---|---|
| *Hnk1* | GAG ACA CCA CGC AAC TAT AA GC | CCTGGCTGAGTAGAGTTCCG |
| *AP2* | CGT CCC GCA CGT AGA AGAC | CGCCACCGAAGAGGTTGTC |
| *Sox10* | AGA AGG AAC AGC AGG ACG GCG A | TGA CGT GCG GCT TGC TCT TG |
| *Sox9* | GTA CCC GCA TCT GCA CAAC | CTCCTCCACGAAGGGTCTCT |
| *P75* | AAA GCC TGC AAC CTG GGC GA | TAG GAG CAT CGG CAC ACG GCA T |
| *Pax3* | ATG GTT GCG TCT CTA AGA TCC TG | GCGTCCTTGAGCAATTTGTC |
| *Na*+/*K*+ *-ATPase* | ATC AGC GAG CTC AGG ACA TT | ACT ACA GCC GCT AGC ACG AT |
| *Aqp1* | CTG GCT CAC GGT GTG AAC TC | CCGCAGCCAGTGTAGTCAAT |
| *slc4a1c* | GGC CAT CGA TGT GCA GAA GA | CGTCCATGCATAGAAGGCGT |
| *Col8a1* | GAC TTC CTGGCATCGGGAAA | CCCATTCCAGGAGCACCAAT |
| *N-cadherin* | CCT TCT GTG TAT CAT CAT CCT | AGT CAT AGT CCT GGT CTT CT |
| *Gapdh* | AACTTTGGCATTGTGGAAGGGCTCA | TTGGCAGCACCAGTGGATGCA GGGA |

### 1.2 RNA-Seq and analysis pipeline

In order to verify the transcriptome analysis of ciNCCs, RNA-seq was performed. RNA-seq libraries were prepared with ovation RNA-seq system v2 kit (NuGEN). The total RNA (50 ng) is reverse-transcribed to synthesize the first-strand cDNA with a combination of random hexamers and a poly-T chimeric primer. The RNA template is then partially degraded by heating and the second-strand cDNA is synthesized using DNA polymerase. The double-stranded DNA is then amplified using single primer isothermal amplification (SPIA). SPIA is a linear cDNA amplification process in which RNase H degrades RNA in DNA/RNA heteroduplex at the 5'-end of the double-stranded DNA, after which the SPIA primer binds to the cDNA and the polymerase starts replication at the 3'-end of the primer by displacement of the existing forward strand. Random hexamers are then used to amplify the second-strand cDNA linearly. Finally, libraries from the SPIA amplified cDNA were made using the Ultralow V2 library kit (NuGEN). The RNA-seq libraries were analyzed by Bioanalyzer and quantified by QPCR (KAPA). Three RNA-seq libraries were pooled per lane of paired-end 100 bp sequencing on HiSeq 2500 instrument (Illumina). Trimming of known adapters and low quality regions of reads was performed using Fastq-mcf. Sample QC was assessed using *FastQC* http://www.bioinformatics.babraham.ac.uk/projects/fastqc/). Reads were aligned to the mouse reference assembly *mm9* using Tophat 2.0.13 (Kim, D., et al. 2011. TopHat 2: accurate alignment of transcriptomes in the presence of insertions, deletions and gene fusions. Genome Biology 14.). Gene-level expression was allied by *Subread featureCounts* (Liao et al. 2014. FeatureCounts: an efficient general-purpose program for assigning sequence reads to genomic features. Bioinformatics 30, 923-930.), using Ensembl's gene annotation for *mm9.* efore processing differential expression, we used RUVSeq to adjust for batch effects (David Risso et al, 2014, Normalization of RNA-seq data using factor analysis of control genes or samples. nature Biotechnology 32, 896-902.). Genes without at least two samples with a CPM (counts per million) value between .5 and 5000 were filtered out. The differential expression P-values were calculated using *edgeR* (Robinson, M.D. et al., 2010. edgeR: a Bioconductor package for differential expression analysis of digital gene expression data. Bioinformatics 26, 139-140). The built-in R function "p.adjust" was used to calculate the FDR using Benjamini-Hochberg method (Benjamini and Hochberg, (1995). Controlling the false discovery rate: a practical and powerful approach to multiple testing. Journal of the Royal Statistical Society Series B 57, 289-300.). Gene ontology analyses were completed through DAVID Bioinformatics Resources 6.7 or ToppGene. Heatmap was generated y Cluster 3.0, and viewed by Java Treeview. Transcriptome analysis revealed that these cells closely resembled mouse NCCs but were distinct from MEFs.

### 1.3 Western blotting

In order to confirm the expression of NC special markers and CEC special markers, western blotting was performed. Briefly, cells were collected in the presence of phosphatase inhibitor (Cell Signaling Technology), and mixed with equal volume of 2×SDS-PAGE samples buffer with DTT. The samples were boiled and cleared by centrifuge. Following electrophoresis, proteins were transferred onto a PVDF membrane. The efficiency of transfer was determined by staining the membrane briefly in Ponceau stain. Membrane was blocked with 5 % BSA at room temperature for at least 1 hour, and incubated with desired antibody diluted in 5 % BSA in TBST at 4 °C overnight. The membrane was washed with TBST for five times (10 min each) before incubated with HRP-conjugated secondary antibody diluted in TBSA at room temperature for 1 hour. After five washing with TBST, the blot was developed by ECL plus detection kit. These results collectively demonstrate that the chemical cocktail has a robust and general effect on reprogramming fibroblasts into ciNCCs and ciCECs (data not shown).

### 2. Maintenance and differentiation potential of ciNCCs

For maintaining ciNCCs after purifying by FACS sorting, cells were cultured on poly-D-lysine/ laminin-coated plates in Neural crest medium consisting of Neurobasal medium (GIBCO) supplemented with 1×N2, 1×B27, 10 µg/ml bFGF, 10 µg/ml EGF, and 10 ng/ml BMP4.

To characterize the differentiation potential of expanded ciNCCs, they were first cultured under differentiation condition. Neuronal differentiation was induced by withdrawal of FGF2/EGF and exposure to BDNF, GDNF, NGF and dibutyryl cyclic AMP (dbcAMP), yielding peripheral neurons. By immunostaining, ciNCCs gave rise to Tuj1+ and Peripherin+ neurons at passage 5, and this differentiation potential was well maintained during prolonged culture (Figure 2A). Schwann cell differentiation, as assessed by the expression of S100b, and GFAP+, was induced in the presence of CNTF, neuregulin 1b and dbcAMP.

For melanocyte differentiation, after 7 days of induction treatment, ciNCCs were cultured in melanocyte differentiation medium (EBM2 basal medium, 5% (v/v) FBS, 100 ng per ml SCF (Life Technology), 200 nM Endothelin 3 (EDN3, Sigma), 50 ng/ml WNT1, 10 ng/ml FGF2, 5µg/ml Insulin, 1 pM cholera toxin, 10 nM 12-O-tetra-decanoylphorbol-13-acetate (TPA, Sigma) and 10µM SB431542 (Sigma). After 3-4 weeks treatment, melanocyte can be observed (Figure. 2A).

To examine differentiation potential toward mesenchymal lineages, we cultured ciNCCs under MSC culture conditions. Under these conditions, cells with mesenchymal morphologies and marker expression (CD105+) emerged (datanot shown). After an additional month of culture, most cells expressed CD105 and a set of surface markers characteristic of mesenchymal stem cell. We used established mesenchymal stem cell differentiation protocols and showed that mesenchymal precursors generated from ciNCCs were capable of adipocytic, chondrogenic and osteogenic (Figure 2B) differentiation.

### 3. Functional characterization of the ciNCC-derived neurons

In order to determine function of the ciNCC-derived neurons, we examined the electrophysiological properties thereof. Whole-cell patch-clamp recordings were taken from ciNCC-derived neurons 10-20 days after co-culturing with rat cortical neurons on differentiation condition. Cultured neurons were transferred to a perfusion stage on an Olympus BX51WI upright microscope and perfused at 2.5 ml/min at room temperature with artificial cerebral spinal fluid (aCSF) containing (in mM): NaCl 119, KCl 2.5, NaH₂PO₄ 1, NaHCOs 26.2 and glucose 11, CaCl₂ 2.5 and MgSO₄ 1.3, with the osmolality adjusted to 300 osm L-i. The aCSF was bubbled with 95% O₂ and 5% CO₂ throughout the recordings. Data were gathered through a MultiClamp 700B amplifier (Axon Instruments), filtered at 2 kHz, and digitized at 10 kHz. Offline analysis was carried out in Igor Pro (Wavemetrics). Action potentials were recorded under the current-clamp whole-cell configuration. The pipette solution for current-clamp experiments contained (in mM): K gluconate 123, KCl 10, MgCl₂ 1, HEPES 10, EGTA 1, CaCl₂ 0.1, K₂ATP 1, Na₄GTP 0.2, and glucose 4, pH adjusted to 7.2 with KOH. Membrane potential was held around -70 mV and step currents were injected of - 20 to 50 pA at 10-pA intervals. Whole-cell currents were recorded at a holding potential of - 70 mV with voltage steps ranging from -70 mV to +30 mV were delivered at 20-mV increments. Spontaneous postsynaptic current were recorded in the whole-cell voltage clamp mode. The whole-cell pipette solution for synaptic current recordings contained (in mM): CsCl 135, HEPES 10, EGTA 1, Mg-ATP 4, Na₄GTP 0.4, and QX-314 10, pH 7.4. To sample the excitatory and inhibitory current, 1 mM glutamate and 100 µM GABA were puffed under 10 p.s.i. for 100 ms, and the holding voltages were -70 mV and 0 mV, respectively. As expected, they generated repetitive trains of action potentials elicited by depolarizing the membrane in the current-clamp mode, indicating that the ciNCC-derived neurons have normal neuron activity (data not shown).

### 4. Cell proliferation assay

In order to measure proliferative capacity of ciCECs, cell proliferation assay were carried out. Proliferation rate of ciCECs cultured in stage II medium was determined by Click-iT^{™} ethynyl deoxyuridine (EdU) Alexa Fluor 488 imaging kit (Invitrogen/Life Technologies) as per the manufacturer's instructions. Briefly, passaged CECs were seeded onto a FNC-coated slide at a lower density of 5 × 10³ cells per cm² and cultured for 24 h. The results indicate these cells have high proliferative capacity (data not shown).

### 5. ciCEC safety verified by teratoma formation test

To evaluate the potential risk of tumorigenesis, 1×10⁶ ciCECs were subcutaneously injected into NOD-SCID mouse and teratoma formed from 4 to 8 weeks. For generation of chimeras, ciCECs were injected into ICR blastocysts and transplanted into pseudopregnant ICR females. The resulting chimeric mice were determined for germline transmission by mating F2 mice with ICR mice. All of the animal experiments were performed with the approval and according to the guidelines of the Animal Care and Use Committee of the Guangzhou Institutes of Biomedicine and Health. No tumors formed over 6 months after transplantation with ciCECs, whereas large teratomas developed in recipients transplanted with mESCs after 4-8 weeks (data not shown). This suggests that ciCECs have minimal, if any, tumorigenic potential capacity. Furthermore, ciCECs maintained a normal karyotype during 30 continuous passages *in vitro* (data not shown). To better understand differentiation *in vivo,* ciCECs were transplanted into the eye of NOD/SCID mice. After 4-8 weeks, the transplanted ciCECs did not formed tumors over 6 months after transplantation with ciCECs (data not shown).

### 6. Engraftment of ciCECs to rabbit injured eyes

To assess whether ciCECs possessed the capacity to engraft and expand *in vivo,* we transplanted them into the rabbit model induced to become bullous keratopathy by mechanically scraping corneal endothelium from the Descemet's membrane. Rabbits were divided into 2 groups (n=10 each group) and the right eye was used for this experiment. The ciCECs were injected into the anterior chamber (Figure 6B 1^{st} image). Each recipient received 1 × 10⁶ (low dose) ciCECs or 2×10⁶ (high dose) ciCECs. Untreated normal (Figure 6B 4^{th} image) and PBS-only-injected (Figure 6B 3^{rd} image) rabbit were used as controls.

After disinfection and sterile draping of the operation site, a 6-mm sclero corneal incision centered at 12 o'clock was made with a slit knife (Alcon Surgical, Shanghai, China), and a viscoelastic agent (Healon; Amersham Pharmacia Biotech AB) was infused into the anterior chamber. After the corneal surface had been ruled with a marking pen (Devon Industries Inc, Madrid, Spain), a 6.0-mm diameter circular Descemet or hex is was created in the center of the cornea with a 30-gauge needle (Terumo, Tokyo, Japan), and Descemet's membrane was removed from the anterior chamber of the eye. The corneal endothelium was mechanically scraped with a lacrimal passage irrigator (Shandong Weigao) from the Descemet's membrane. Fsp-ciCECs were dissociated using 0.25% trypsin-EDTA and resuspended in PBS at 1 × 10⁷ cells/ml density and kept on ice. The anterior chamber was washed with PBS three times.

After this procedure, a 26-gauge needle was used to inject 1×10⁶ ciCECs suspended in 100 µl of basic-DMEM medium containing 100 µM ROCK inhibitor Y-27632 (which promotes cell adhesion to the implantation sites) (ROCK inhibitor, Selleck) into the anterior chamber of the right eye (Figure 6B, 1^{st} image).

After the procedure, the rabbits were placed in a prone position for 2-3 hours. Each surgical eye was checked twice or three times a week by external examination, and photographs were taken on days 1, 3, 5, 7, 14, 21, 35 and 42 after surgery. Slit-lamp photographs showed that the clarity of the CEC-like cell group (ciCECs group) corneas significantly improved after injection, and the pupil and iris texture could be seen. After only about 7 days, the corneas became clearly transparent, while corneal opacity and stromal edema were still serious in the control group (Figure 6B 1^{st} and 3^{rd} images from the left, respectively). Visante OCT also showed that the corneal thickness rapidly decreased after injection of CEC-like cells (Figure. 6C, each image corresponds to the above images of Figure 6B, respectively). Confocal microscope images confirmed full coverage of polygonal cells on the Descemet's membrane in the ciCEC group (Figure 6D, 1^{st} image. Each image in Figure 6D corresponds to the above images of Figure 6B and Figure 6C, respectively). It could be seen that the mean corneal thickness on 1, 3, 5, 7, 14, 21, 35 and 42 days in the ciCEC group were significantly less than that in the untreated control group (Figure 6E). Slit-lamp photographs showed the clarity of the ciCEC group corneas significantly improved on 1, 3, 7, 14, 21, and 28 days (Figure 7).

### EXAMPLE 4: GENERATION OF CINCCS AND CICECS FROM FIBROBLASTS ACCORDING TO ANOTHER EXAMPLE

### M6 reprogramming medium preparation

The basal medium contained knockout DMEM (Gibco), 10% KSR (Gibco), 10% FBS (Gibco), 1% NEAA (Gibco), and 0.1 mM 2-mercaptoethanol (Gibco) supplemented with the small molecules CHIR99021 (3 µM), SB431542 (5 µM), Forskolin (10 µM), VPA (500 mM), EPZ004777 (5 µM), and 5-Aza-dC (0.5 µM). The medium was shaken for 30 min to ensure that all components were fully dissolved.

### Differentiation medium preparation

DMEM/F12/GlutaMAX (Gibco), 10% KSR (Gibco), 1% NEAA (Gibco), and 0.1 mM 2-mercaptoethanol (Gibco) were supplemented with SB431542 (5 µM) and CKI-7 (5 µM).

### Chemical conversion of NCCs from fibroblasts

The MEFs were plated at 5×10⁴ cells per well on six-well tissue culture plates in fibroblast medium. The culture plates were precoated with fibronectin or laminin for more than 2 hours. After overnight culture, the medium was exchanged with M6 chemical medium, which was refreshed every 2 days. NCC-like cells appeared and increased at days 3 to 5. After 7 to 10 days of induction, FACS was performed to collect Wnt1⁺ cells.

### Chemical induction of CEC-like cells from mouse ciNCCs

On days 12 to 16, the M6 chemical medium was replaced with SB431542 and CKI-7 medium, which was refreshed every 2 days. Endothelial-like cell clusters appeared on day 8 and increased, Oct4-GFP-positive clusters appeared on day 12 and CEC-like cells appeared as early as day 20. During days 30 to 35, these CEC-like cell colonies were counted or further detected.

### ciNCC differentiation

Approximately 5×10³ ciNCCs were seeded on laminin-coated slides in 24-well tissue culture plates and cultured in NCSC medium for the first 24-hour. After 24 hours, cells were subjected to differentiation conditions. For neuron differentiation, the medium was switched to neuron differentiation medium [NCC medium without bFGF and EGF, with the addition of 200 µM ascorbic acid, 2 µM dibutyryl cAMP (db-cAMP), 25 ng/ml brain-derived neurotrophic factor (BDNF), 25 ng/ml NT3, and 50 ng/ml glial cell line-derived neurotrophic factor (GDNF)]. Half of the medium was changed every 2 to 3 days. Specific neuron markers were analyzed by day 10 to day 20 after differentiation. To differentiate into melanocytes, cells were cultured in the presence of 5 µM retinoic acid (RA) and Shh (Sonic hedgehog) (200 ng/ml) for 1 day and platelet-derived growth factor-AA (PDGF-AA) (20 ng/ml), bFGF (20 ng/ml), and Shh (200 ng/ml) for 3 to 5 days; then, they were cultured in differentiation medium containing T3 (40 ng/ml), Shh (200 ng/ml), 1 nM LDN193189, 5 mM db-cAMP, and NT3 (10 ng/ml) for 8 to 12 days. The medium was refreshed every other day. For astrocytes differentiation, BMP4 (50 ng/ml) was added into the differentiation medium for 8-12 days. The medium was refreshed every other day.

### Results

For corneal endothelium is derived from NCCs, a two-step method for reprogramming mouse fibroblasts into CEC-like cells using small molecules was designed, in which the first step was to guide mouse embryonic fibroblasts (MEFs) to be chemically reprogrammed into ciNCCs. To identify small molecules with the potential to convert fibroblasts into ciNCCs, we used a lineage tracing strategy to trace the conversion process and excluded any NCCs or progenitor cells from the starting MEFs (Figures 8A and 15A). Wnt1-Cre transgenic mice have been validated as a lineage-tracing reporter model for NCC development. In Wnt1-Cre/ROSA26tdTomato mice, tdTomato protein was faithfully expressed in NCCs. MEFs were thus isolated from Wnt1-Cre/ROSA26tdTomato mice at embryonic day 13.5 (E13.5). Because the NCC population was marked with tdTomato, we performed fluorescence-activated cell sorting (FACS) to collect the tdTomato⁻ population to exclude any NCCs or progenitors (the purified cells are hereinafter referred to as Wnt1-tdTomato⁻MEFs; Figure 15B). We confirmed that the Wnt1-tdTomato⁻ MEFs were also negative for other NCC markers, including Sox10, P75, Pax3, HNK1, and AP2α (Figures 15C and 15D). Further, these Wnt1-tdTomato⁻ MEFs were negative for typical NSC markers, including Sox2, Pax6 and Nestin (data not shown).

It is reported that some small molecules for enhancing reprogramming can promote lineage reprogramming. To generate ciNCCs from MEFs, we selected 16 small molecules as candidates based on (1) the epigenetic regulation and signaling modulation of NCC development and (2) enhanced neural lineage reprogramming. The small molecules for NC lineage reprogramming were initially focused on three categories of reprogramming factors comprised of Chir99021 (a GSK3 inhibitor), SB431542 (a TGF-β inhibitor) and forskolin (a cAMP inducer) (Figure 8B). For subsequent screening and optimization, we found VPA (a HDAC inhibitor), EPZ004777 (a DOT1L inhibitor) and 5- Aza-dC (a DNA methyltransferase inhibitor) further promoted induction of Wnt1-tdTomato⁺ cells (Figure 8C). In this study, we used a chemically defined medium combined with a cocktail of the above-mentioned six small molecules (hereafter termed M6) to reprogram MEFs into ciNCCs (Figure 8D). Expression of Wnt1-tdTomato in individual cells was observed as early as day 3 with M6 medium treatment (Figure 8E). The M6 reprogramming medium effectively induced Wnt1-tdTomato⁺ cells at 3.97% (Figure 8F). On days 5 to 7, inducted Wnt1-tdTomato⁺ colonies were observed in the M6 reprogramming medium (Figures 8G and 15E). These Wnt1-tdTomato⁺ cells and colonies had molecular feature similar to those of primary NCCs (pNCCs). The Wnt1-tdTomato⁺ cell number increased notably in small colonies at approximately day 12 (Figure 8H). Although the generation of Wnt1-tdTomato⁺ NCCs was only as efficient as the conversion of human fibroblasts using TFs, merely 2 to 5% of cells were positive for Wnt1-tdTomato in this study. The results were reproducible in different batches of MEFs (n = 8), and MEFs with different genetic backgrounds (C57BL/6, 129×C57BL/6, and 129) could also be converted into ciNCCs via M6 conditions. Together, these results indicate that M6 can reprogram MEFs into ciNCCs.

### EXAMPLE 5: CHARACTERIZATION OF CINCCS AND CICECS OBTAINED FROM EXAMPLE 4 AND ADDITIONAL INVESTIGATION

### Characterization of the converted ciNCCs

There are two stages in the ciNCCs reprogramming process: the initial stage (days 0-7) and the amplification stage (days 7-12). The first stage is to culture MEFs in a reprogramming medium to initiate epigenetic activation, where a few small NCC-like clusters with clear-cut edges emerge. In the second stage, epigenetic activated cells are cultured in a small-molecule-defined medium, in which most clusters expand and grow gradually.

To obtain the ciNCCs, we performed FACS to collect the Wnt1-tdTomato⁺ cells. Established ciNCCs were serially propagated in NCC expansion medium containing N2, B27, bFGF, and EGF. Morphologically, M6-induced cells at P3 maintained typical NCC features in monolayer culture (Figure 9A). After passaging, the ciNCCs became morphologically homogeneous. To further characterize the M6-induced Wnt1-tdTomato⁺ cells, we sought to examine their gene expression. Our results showed the ciNCCs expressed multiple NCC markers, including P75, HNK1, AP2, and Nestin (Figure 9B). Furthermore, we tested if the ciNCCs have differentiation potential toward peripheral neurons, Schwann cells, and others. For differentiation of ciNCCs, these cells were cultured in different lineage differentiation media. After cultured for 2 to 4 weeks, the differentiated cells were examined by assessing the expression of the markers by immunostaining. Notably, the ciNCCs could also give rise to cells expressing specific markers for neuron, including Tuj1 and Peripherin (Figure 9C). For melanocyte differentiation, we observed melanocytes after 2 to 3 weeks of induction (Figure 9C). Our results of immunostaining showed that the ciNCCs could differentiate to Schwann cells. The induced Schwann cells were GFAP⁺ and S100⁺ cells (Figure 9D). Further differentiation of these ciNCCs *in vitro* gave rise to mesenchymal lineages, resulting in typical mesenchymal cell morphology. Our results showed that these ciNCC-derived mesenchymal cells could give rise into osteogenic, adipogenic, and chondrogenic cells (Figure 9E). Collectively, these data indicated that our ciNCCs could be induced to differentiate toward peripheral nervous system lineages and mesenchymal lineages.

### Transplantation into an animal model revealed ciCEC function

To evaluate whether the ciCECs had the capacity to regenerate the corneal endothelia *in vivo*, we transplanted them into a well-established rabbit model with bullous keratopathy by mechanically scraping corneal endothelia from Descemet's membrane. A previous study found that injection of human pCECs supplemented with a ROCK inhibitor restored endothelial function. In this study, the ciCECs were injected combined with a ROCK inhibitor (Y-27632) into the anterior chambers of the eyes (Figure 14A). Each recipient received 1 × 10⁶ ciCECs. Contralateral eyes (normal) and untreated eyes (PBS injection) were used as experimental controls. Corneal edema decreased much earlier after ciCEC transplantation in the grafted eyes than in the untreated eyes. Compared to the untreated eyes, which showed that the clarity of the cornea was unchanged, we noted that the clarity of the cornea of the grafted eyes increased gradually after transplantation (Figures 14B and 19A). After 7 days, the corneas of the grafted eyes became transparent, while corneal opacity and stromal oedema remained poor in the untreated eyes. The results of slit lamp examination showed that the clarity of the cornea of the grafted eyes was also notably improved after injection, and the pupil and iris texture could be clearly observed (Figures 14C and 19A).

Next, we investigated ciCEC survival in the grafted eyes. Eyeballs were enucleated at postsurgical day 28 to evaluate the transplanted ciCECs. Fluorescence microscopy examination confirmed the existence of tdTomato-tagged grafted cells. Immunohistochemistry demonstrated ZO-1 expression, indicating a pump function of the transplanted ciCECs (Figures 14D and 19C).

Visante optical coherence tomography (OCT) of the anterior segment also showed that the corneal thickness decreased after ciCEC injection (Figure 14E). Confocal microscopy confirmed full coverage of polygonal endothelial cells on Descemet's membrane in the ciCEC-grafted disease models, while this could not be detected in the untreated models due to their severe corneal opacity (Figure 14G). Under magnification, the grafted ciCECs tightly adhered to the posterior surface of the cornea in a monolayer, whereas the Descemet's membranes in the untreated model were bare and had no detectable CECs on day 28.

There was a rapid decrease in corneal thickness within 4 weeks after ciCEC injection, followed by a more gradual decrease over the next 2 weeks (Figures 14F and 19D). In the untreated group, mean corneal thickness was approximately 1200 µm throughout the 42 days of observation. In contrast, it decreased rapidly in the grafted group, being significantly less than that in the untreated group. We observed that the mean corneal thickness at postoperative days 14 (P < 0.01), 21, 28, 35, and 42 (P < 0.001) in the ciCEC-grafted group was significantly less than that in the control group, indicating that corneal edema was markedly reversed. These results strongly suggest that ciCEC transplantation repopulated and self-organized on the posterior surface of the cornea and has the capacity to regenerate the corneal endothelium.

### Small molecules facilitate the induction of ciNCCs into ciCECs

To generate mouse CEC-like cells from ciNCCs, we sought small molecules based on their importance in CEC organogenesis and maintenance *in vitro.* In the initial screening, we found SB431542 and CKI-7 were capable of inducing CEC-like cells from ciNCCs. These two compounds were then included in the differentiation conditions (Figure 10A). To determine whether ciNCCs can be further differentiated into mature CECs, we used a differentiation medium containing these two small molecules to treat ciNCCs. After 7 to 15 days of culture in this differentiation medium, a small population outside or inside the clusters displayed typical tight aggregates with homogeneous and polygonal morphology (Figure 10B). We also observed that these colonies rapidly expanded, and the small clusters merged into larger ones by days 12 to 15 (Figure 10B). These CEC-like cells grew rapidly and had strong proliferation ability. The ciNCC-derived CEC-like cells formed a monolayer of hexagonal and pentagonal cells. To confirm that the CEC-like cells were derived from ciNCCs, we differentiated tdTomato⁺ ciNCCs with CEC differentiation medium containing 5µM SB431542 and 5µM CKI-7. These tdTomato⁺ ciNCCs could also be subsequently induced to differentiate toward CEC-like cells (Figure 10C). The expression of Na⁺/K⁺-ATPase, AQP1, vimentin, ZO-1, and N-cadherin was further verified by immunofluorescence staining (Figure 10D). In this study, we identified the function of the CEC-like cells by Dil-labeled acetylated low-density lipoprotein (Dil-Ac-LDL) uptake (Figure 10E). Global gene expression analysis by RNA sequencing (RNA-seq) showed that the CEC-like cells shared a similar gene expression pattern to that of primary CECs (pCECs), but this pattern was distinct from that of the initial MEFs (Figure 10F). The presence of tight junctions in the CEC-like cells was observed by transmission electron microscopy (TEM) (Figure 10G). To further monitor the reprogramming process, we confirmed the expression of a panel of NCC and CEC markers at distinct times by using quantitative reverse transcription polymerase chain reaction (qRT-PCR). By day 12, robust expression of NCC genes was detected in cells, including Hnk1, P75, Sox10, Sox9, Pax3, and Ap2 (Figure 11A). In addition, similar kinetics of gene activation for CEC genes, such as Slc4alc, Col8a1, Na⁺/K⁺-ATPase, Aqp1, and N-cadherin, were also detected by qRT-PCR (Figure 11B). The genes that are known to be enriched in pCECs were greatly up-regulated in ciCECs. To validate the transition process from fibroblast to CECs, we analyzed the transcriptome by using RNA-seq (Figure 11C). The expression of CEC signature genes in ciCECs was substantially up-regulated, consistent with that of pCECs, whereas fibroblast signature genes were markedly down-regulated. Notably, a panel of NCC marker genes was initially up-regulated and subsequently down-regulated during the induction process. Principal components analysis revealed that M6-treated cells were distinct from the original MEFs, indicating that chemical reprogramming led to marked transcriptional changes (Figure 11D). These results indicate that the CEC-like cells obtained CEC identity. Collectively, these data suggest that the combination of SB431542 and CKI-7 effectively promoted the generation of CECs within 10 to 15 days in the ciNCC culture. Those fibroblast-originated CEC-like cells are thereafter referred to as chemically induced CEC-like cells (ciCECs).

### Lineage tracing to confirm the induction of ciCECs from fibroblasts

To confirm the origin of the initial fibroblasts for the small molecule-based reprogramming, we sought a genetic lineage-tracing strategy to purify fibroblast-specific protein 1 (Fsp1)-tdTomato⁺ fibroblasts (Figure 12A). Fsp1-Cre has been validated as a specific fibroblast marker for lineage tracing; thus, Fsp1-Cre mice were crossed with ROSA26tdTomato mice. MEFs were isolated from transgenic mice (Fsp1-Cre/ROSA26tdTomato) at E13.5, and the fibroblasts expressed tdTomato specifically; these cells are hereafter named tdMEFs (Figure 16A). To avoid possible contamination of the MEFs with NCC progenitor cells, we carried out FACS to collect the tdTomato⁺/p75⁻ population (Figure 12B). These tdMEFs were negative for all NCC markers, including P75, HNK1, Sox10, and AP2 (Figure 12C).

Then, these tdMEFs were induced with the abovementioned M6 medium. Epithelial clusters expressing tdTomato were observed during ciNCC induction (Figures 12D and 16B). We passaged the Fsp1-tdTomato⁺ ciNCCs and cultured them in NCC expansion medium for further experiments (after 2 weeks of induction). Immunofluorescence analysis confirmed that these Fsp1-tdTomato⁺ ciNCCs were positive for the NC markers P75, HNK1, Sox10, and AP2α, indicating that the colonies of Fsp1-tdTomato⁺ ciNCCs had differentiated toward CEC-like cells (Figure 16C). Furthermore, the Fsp1-tdTomato⁺ ciNCCs could differentiate into tdTomato⁺ ciCECs (Figure 16D). By immunostaining, we found that differentiated CEC-like cells coexpressed Na⁺/K⁺-ATPase, AQP1, laminin, ZO-1, Na⁺/K⁺-ATPase, and tdTomato (Figure 12E). Notably, all these ciCECs also expressed tdTomato, demonstrating a conversion from fibroblasts. The results confirmed that the ciNCCs and ciCECs were converted by two-step lineage reprogramming from fibroblasts.

### ciCECs generated with chemicals bypass the iPSC stage

Because the mechanism of our lineage reprogramming might be similar to that of chemically reprogrammed iPSCs, we sought to assess whether the ciCECs went through an iPSC stage. We performed a comparison between chemical iPSC reprogramming and ciCEC induction from MEFs derived from mice harboring an Oct4 promoter-driven green fluorescent protein (GFP) (OG2) reporter. We observed the M6-treated MEFs morphologically underwent a characteristic mesenchymal-epithelial transition (MET), and small cell colonies gradually emerged toward day 6 (Figure 17A). These cell colonies expressed the NCC marker Sox10 (Figure 17B). In contrast, we did not observe any Oct4-GFP-positive cells during the entire process from MEFs to ciCECs based on our method (Figure 17C and 3D). Furthermore, the ciCECs maintained a normal karyotype during 10 continuous passages *in vitro* (Figure 17E). To evaluate the potential risk of tumorigenesis, a total of 5×10° ciCECs and 2×10° mouse embryonic stem cells (mESCs) were transplanted subcutaneously into nonobese diabetic mice (NOD)/severe combined immunodeficient (SCID) mice. Notably, no tumors formed over 6 months after transplantation with ciCECs, whereas large teratomas developed in the mice transplanted with mESCs after 4 to 8 weeks (data not shown). This result suggests that the ciCECs have no tumorigenic potential. To better understand their differentiation *in vivo*, we transplanted ciCECs into the anterior chambers of the eyes of NOD/SCID mice. After 4 to 8 weeks, the transplanted ciCECs did not form tumors over 6 months after transplantation. These results demonstrated that our approach could directly reprogram MEFs to ciNCCs and eventually ciCECs while bypassing the iPSC stage.

### Long-term expansion capacity of ciCECs in vitro

The maintenance of cultured CECs with morphology and normal physiological function *in vitro* has proven challenging. We aimed to test whether large numbers of functional ciCECs could be generated from fibroblasts to enable the large-scale application of ciCECs. On the basis of our observations that ciCECs cultured in medium with SB431542 (5 µM) and CKI-7 (5 µM) were small, polygonally shaped cells (no epithelial- mesenchymal transition like cell) (Figure 18A), we hypothesized that SB431542 and CKI-7 would promote ciCEC growth *in vitro.* We evaluated the long-term expansion capacity of ciCECs *in vitro* by continuously passaging ciCECs at a 1:6 ratio and found that the phenotype was similar between P3 and P30 (Figures 13A and 13B). This result showed that SB431542 and CKI-7 strongly promoted ciCEC expansion. These ciCECs sustained themselves as a homogeneous cell population for at least 30 passages (P30) with hexagonal morphology in small molecule-based medium. In addition, we successfully clonogenically cultured these ciCECs to 10 passages and demonstrated consistent morphologies. Our results of immunostaining showed that the rate of Ki67-positive cells was higher in ciCECs at P3 than in pCECs at P3 (Figure 18B). ciCECs were highly proliferative, as 24.6%, 37.8%, and 48.1% of these cells showed incorporation of ethynyl deoxyuridine (EdU) at P1, P3, and P6, respectively (Figure 18C). The results of FACS analysis with propidium iodide (PI) staining showed that the cell cycle distribution (G0/G1, S and G2/M phases) was 46.30%, 45.11%, and 8.59% for ciCECs at P3 and 67.30%, 21.50%, and 11.20% for pCECs at P3 (Figure 18D). They rapidly expanded into large, homogeneous colonies with population doubling times of 22.3 ± 3.7 hours (Figure 13C). Interestingly, large "vacuole-like" structures were found on the surface of the ciCECs at P2 to P10 (Figure 13D). These vacuole-like structures disappeared at P20 when they were serially propagated. Notably, the ciCECs at P30 also expressed typical CEC markers, including Na⁺/K⁺-ATPase, AQP1, and ZO-1 (Figure 13E). When assayed by imaging for the ability to migrate into the space created by a scratch wound, ciCECs cultured in medium with SB431542 and CKI-7 at different passages showed a stronger capability of proliferation and migration as compared to that of pCECs (Figure 18E and 18F). Collectively, these results demonstrated that SB431542 and CKI-7 had a robust and general effect on long-term expansion of ciCECs *in vitro.*

## Claims

1. A method for reprogramming a first type of cells into a second type of cells, comprising culturing the first type of cells in the presence of a first group of reprogramming factors, wherein the first group of reprogramming factors comprises a glycogen synthases kinase 3 (GSK3) inhibitor, a transforming growth factor-β (TGFP) inhibitor, and a cyclic AMP inducer.

2. The method of claim 1, wherein the first group of reprogramming factors further comprises a basic fibroblast growth factor (bFGF), a DNA methyltransferase inhibitor, a DOT1L inhibitor, a histone deacetylase inhibitor, BMP4, or a combination thereof.

3. The method of claim 1, wherein the first group of reprogramming factors consists of (a) a GSK3 inhibitor, a TGFβ inhibitor, and a cyclic AMP inducer, (b) a GSK3 inhibitor, a TGFβ inhibitor, a cyclic AMP inducer and bFGF, or (c) a GSK3 inhibitor, a TGFβ inhibitor, a cyclic AMP inducer, a DNA methyltransferase inhibitor, a DOT1L inhibitor and a histone deacetylase inhibitor.

4. The method of claim 1, wherein the GSK3 inhibitor is selected from a group consisting of CHIR99021, LiCl, Li₂CO₃, BIO ((2'Z, 3'E)-6-Bromoindirubin-3'-oxime), TD114-2, Kenpaullone, TWS119, CBM1078, SB216763, 3F8(TOCRIS) , AR-A014418, FRATide, indirubin-3'-oxime and L803.

5. The method of claim 1, wherein the TGFP inhibitor is selected from a group consisting of SB431542, Repsox, 616452, LDN193189, A8301, GW788388, SD208, SB525334, LY364947, D4476, SB505124,and Tranilast.

6. The method of claim 1, wherein the cyclic AMP inducer is forskolin, IBMX, Rolipram, 8BrcAMP, Prostaglandin E2 (PGE2), NKH477, Dibutyryl Monocyclic Adenylic Acid (DBcAMP), and Sp-8-Br-cAMPs.

7. The method of claim 2, wherein the DNA methyltransferase inhibitor is selected from a group consisting of 5-aza-dC, 5-azacytidine, and RG108.

8. The method of claim 2, wherein the DOT1L inhibitor is EPZ004777.

9. The method of claim 2, wherein the histone deacetylase inhibitor is selected from a group consisting of Valproic acid (VPA), trichostatin A (TSA), vorinostat, depsipeptide, Trapoxin, Depudecin, FR901228, and butyrate.

10. The method of claim 1, wherein the first type of cells is somatic cells.

11. The method of claim 10, wherein the somatic cells are derived from mesoderm, ectoderm or endoderm.

12. The method of claim 10, wherein the somatic cells are fibroblasts.

13. The method of claim 12, wherein the fibroblasts is selected from a group consisting of mouse embryonic fibroblasts (MEFs), mouse tail tip fibroblasts (TTFs), human embryonic fibroblasts (HEFs), human neonatal fibroblasts (HNFs), human adult fibroblasts (HAFs), human foreskin fibroblasts (HFFs), and the mixture thereof.

14. The method of claim 10, wherein the somatic cells are human exfoliated renal epithelial cells.

15. The method of claim 1, wherein the first type of cells is stem cells.

16. The method of claim 15, wherein the stem cells are selected from a group consisting of human umbilical cord mesenchymal stem cells, human embryonic stem cells, and induced pluripotent stem cells (iPSCs).

17. The method of claim 1, wherein the second type of cells is stem cells.

18. The method of claim 17, wherein the stem cells are neural-crest-like cells (NCC-like cells).

19. The method of claim 18, wherein the NCC-like cells are positive for P75, Hnk1, AP2α, and Sox10.

20. The method of claim 1, wherein the first type of cells is cultured in the presence of the first group of reprogramming factors for (a) at least 5, 6, 7, 8, 9, 10, 11, or 12 days or (b) no more than 20, 19, 18, 17, 16, 15, 14, 13, or 12 days.

21. A method for reprogramming a second type of cells into a third type of cells, comprising culturing the second type of cells in the presence of a second group of reprogramming factors, wherein the second group of reprogramming factors comprises a TGFβ inhibitor and a casein kinase 1 inhibitor.

22. The method of claim 21, wherein the second group of reprogramming factors further comprises BMP4 and/or a DNA methyltransferase inhibitor.

23. The method of claim 21, wherein the casein kinase 1 inhibitor is CKI-7.

24. The method of claim 22, wherein the DNA methyltransferase inhibitor is selected from a group consisting of 5-aza-dC, 5-Azacytidine, and RG108.

25. The method of claim 21, wherein the third type of cells is somatic cells.

26. The method of claim 25, wherein the somatic cells are corneal endothelial cell (CEC)-like cells.

27. The method of claim 26, wherein the CEC-like cells are positive for ZO-1 and Na⁺/K⁺-ATPase.

28. The method of claim 21, wherein the second type of cells is cultured in the presence of the second group of reprogramming factors for (a) at least 5, 6, 7, 8, 9, 10, 11, or 12 days or (b) no more than 20, 19, 18, 17, 16, 15, 14, 13, or 12 days.

29. A method of reprogramming a first type of cells into a third type of cells, comprising step (a) culturing the first type cells in the presence of a first group of reprogramming factors, wherein the first group of reprogramming factors comprises a glycogen synthases kinase 3 (GSK3) inhibitor, a transforming growth factor (TGFβ) inhibitor, and a cyclic AMP inducer, and step (b) culturing cells obtained from step (a) in the presence of a second group of reprogramming factors, wherein the second group of reprogramming factors comprises a TGFβ inhibitor and a casein kinase 1 inhibitor.

30. The method of claim 29, wherein the first group of reprogramming factors further comprises a DNA methyltransferase inhibitor, a DOT1L inhibitor, and a histone deacetylase inhibitor.

31. The method of claim 29, further comprising washing cells obtained from step (a) before starting step (b).

32. The method of claim 29, wherein there is no washing step between step (a) and step (b).

33. A method of reprogramming a first type of cells into a third type of cells, comprising culturing the first type of cells in the presence of a first group of reprogramming factors and a second group of reprogramming factors, wherein the first group of reprogramming factors comprises a glycogen synthases kinase 3 (GSK3) inhibitor, a transforming growth factor (TGFP) inhibitor, and a cyclic AMP inducer, and the second group of reprogramming factors comprises a TGFβ inhibitor and a casein kinase 1 inhibitor.

34. The method of claim 33, wherein the first group of reprogramming factors further comprises a DNA methyltransferase inhibitor, a DOT1L inhibitor, and a histone deacetylase inhibitor.

35. A population of NCC-like cells produced according to the method of any one of claims 1-20.

36. A population of corneal endothelial cell-like cells (CEC-like cells) produced according to the method of any one of claims 21-34.

37. A composition comprising the NCC-like cells of claim 35 or CEC-like cells of claim 36.

38. A method of treatment of a disease or condition associated with dysfunctional or injured corneal endothelial cells, comprising administrating an effective amount of the CEC-like cells of claim 36 or the composition of claim 37 to a subject in need thereof.

39. The method of claim 38, wherein the subject is human.

40. The method of claim 38, wherein the disease or condition is selected from a group consisting of Fuch's dystrophy, iridocorneal endothelial syndrome, posterior polymorphous dystrophy, congenital hereditary endothelial dystrophy, age-related macular degeneration (AMD), retinitis pigmentosa, glaucoma, corneal dystrophies, contact lens usage, cataract surgery, and late endothelial failure in cornea transplantation.

41. A kit for reprograming a first type of cells into a second type of cells, wherein the kit comprises a first group of reprogramming factors, and the first group of reprogramming factors comprises a glycogen synthases kinase 3 (GSK3) inhibitor, a TGFP inhibitor, and a cyclic AMP inducer.

42. The kit of claim 41, wherein the first group of reprogramming factors further comprises a DNA methyltransferase inhibitor, a DOT1L inhibitor, and a histone deacetylase inhibitor.

43. A kit for reprograming a second type of cells into a third type of cells, wherein the kit comprises a second group of reprogramming factors and the second group of reprogramming factors comprises a TGFP inhibitor and a casein kinase 1 inhibitor.

44. A kit for reprograming a first type of cells into a third type of cells, wherein the kit comprises a first group of reprogramming factors and a second group of reprogramming factors, wherein the first group of reprogramming factors comprises a glycogen synthases kinase 3 (GSK3) inhibitor, a TGFβ inhibitor, and a cyclic AMP inducer, and the second group of reprogramming factors comprises a TGFP inhibitor and a casein kinase 1 inhibitor.

45. The kit of claim 44, wherein the first group of reprogramming factors further comprises a DNA methyltransferase inhibitor, a DOT1L inhibitor, and a histone deacetylase inhibitor.

46. A method of identifying a drug, comprising administering a drug candidate to the NCC-like cells of claim 35 or CEC-like cells of claim 36, and detecting a response of the cells to the drug candidate, thereby identifying the drug.
